Europäisches Patentamt
European Patent Office
Office européen des brevets

(19)

(11) Veröffentlichungsnummer: **0 222 702 A2**

(12) # EUROPÄISCHE PATENTANMELDUNG

(21) Anmeldenummer: **86810492.8**

(22) Anmeldetag: **31.10.86**

(51) Int. Cl.[4]: **C 07 D 211/90**
C 07 D 409/12, A 61 K 31/445

(30) Priorität: **06.11.85 CH 4759/85**

(43) Veröffentlichungstag der Anmeldung:
**20.05.87 Patentblatt 87/21**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Kühnis, Hans, Dr.**
**Lange Gasse 26**
**CH-4052 Basel (CH)**

(54) **Basische Carbonylverbindungen.**

(57) Verbindungen der Formel

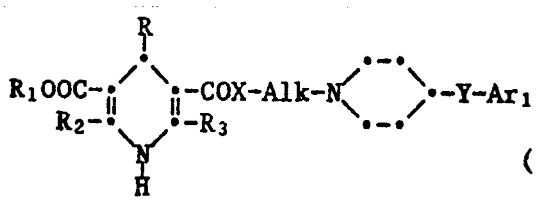

(I)

worin R einen carbocyclischen oder heterocyclischen Arylrest bedeutet, $R_1$ Niederalkyl darstellt, eine der Gruppen $R_2$ und $R_3$ für Niederalkyl und die andere für Niederalkyl, Cyano oder Amino steht, X Sauerstoff oder die Gruppe -NH- darstellt, und Alk durch ein carbocyclisches Aryl substituiertes Alkylen bedeutet, das die Gruppe X vom Ringstickstoffatom durch mindestens zwei Kohlenstoffatome trennt, Y für Alkylen oder die Gruppen -CH(OH)- oder -C(=O)-steht oder eine einfache Bindung bedeutet, und $Ar_1$ einen monocyclischen Aryl- oder Heteroarylrest darstellt, und ihre Salze können als Coronardilatatoren und Antihypertensiva verwendet werden und werden in an sich bekannter Weise hergestellt.

EP 0 222 702 A2

**Beschreibung**

Basische Carbonylverbindungen

Die Erfindung betrifft neue basische Carbonylverbindungen und deren Salze, Verfahren zu ihrer Herstellung, diese enthaltende pharmazeutische Präparate sowie deren Verwendung.

Die erfindungsgemässen Verbindungen entsprechend der Formel

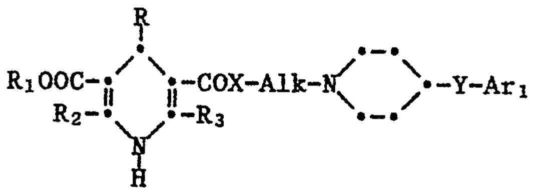

(I),

worin R einen carbocyclischen oder heterocyclischen Arylrest bedeutet, $R_1$ Niederalkyl darstellt, eine der Gruppen $R_2$ und $R_3$ für Niederalkyl und die andere für Niederalkyl, Cyano oder Amino steht, X Sauerstoff oder die Gruppe -NH- darstellt, und Alk durch ein carbocyclisches Aryl substituiertes Alkylen bedeutet, das die Gruppe X vom Ringstickstoffatom durch mindestens zwei Kohlenstoffatome trennt, Y für Alkylen oder die Gruppen -CH(OH)- oder -C(=O)-steht oder eine einfache Bindung bedeutet, und $Ar_1$ einen monocyclischen Aryl- oder Heteroarylrest darstellt, und umfassen Salze von Verbindungen der Formel I.

Ein carbocyclischer oder heterocyclisher Arylrest R ist in erster Linie ein entsprechender monocyclischer Rest, kann jedoch auch ein bi- oder polycyclischer, carbocyclischer oder heterocyclischer Rest mit aromatischen Eigenschaften sein.

Carbocyclische Reste R dieser Art sowie der als Substituent in einer Alkylengruppe Alk enthaltene carbocyclische Rest sind insbesondere gegebenenfalls substituiertes Phenyl, ferner Naphthyl.

Heterocyclische Arylrest R sind vorzugsweise entsprechende monocyclische Reste, können jedoch auch entsprechende bi- oder polycyclische Reste sein, wobei letztere aus mehreren heterocyclischen Ringe, oder aus einem oder mehreren Ringen mit einem oder mehreren ankondensierten carbocyclischen Ringen, insbesondere einem oder mehreren ankondensierten Benzoringen bestehen können. Die üblicherweise vorliegenden heterocyclischen Reste R, die vorzugsweise aus fünf oder sechs Ringgliedern bestehen, können bis zu vier, gleiche oder verschiedene Hetero-, insbesondere Stickstoff-, Sauerstoff-und/oder Schwefelatome, vorzugsweise ein, zwei, drei oder vier Stickstoffatome, ein Sauerstoff- oder Schwefelatom, oder ein oder zwei Stickstoffatome zusammen mit einem Sauerstoff- oder Schwefelatom als Ringglieder enthalten. Sie sind mit dem Kohlenstoffatom der 4-Stellung des 1,4-Dihydropyridinringes üblicherweise über ein Ringkohlenstoffatom gebunden.

Monocyclische fünfgliedrige Heteroarylreste R sind z.B. entsprechende monoaza-, diaza-, triaza-, tetraza-, monooxa-, monothia-, oxaza-, oxadiaza-, thiaza- oder thiadiazacyclische Reste, wie Pyrryl-, Pyrazolyl-, Imidazolyl-, Triazolyl-, Tetrazolyl-, Furyl-, Thienyl-, Isoxazolyl-, Oxazolyl-, Oxadiazolyl-, Isothiazolyl-, Thiazolyl- oder Thiadiazolylreste, während monocyclische, sechsgliedrige Heteroarylreste R z.B. entsprechende monoaza-, diaza- oder triazacyclische Reste, wie Pyridyl-, Pyridazinyl-, Pyrimidinyl-, Pyrazinyl- oder Triazinylreste sind. Bicyclische Heteroarylreste sind insbesondere monocyclische Heteroarylreste mit ankondensiertem Benzoring; der Heteroring kann fünf- oder sechsgliedrig sein, wobei ein fünfgliedriger Heteroarylrest z.B. einen monoaza-, diaza-, monooxa-, monothia-, oxaza- oder thiazacyclischen Rest, und ein sechsgliedriger Heteroarylrest z.B. einen monoaza- oder einen diazacyclischen Heteroarylrest darstellt. Solche bicyclischen Reste, die über ein Ringkohlenstoffatom des hetero- oder carbocyclischen Restes gebunden sein können, sind z.B. Indolyl-, Isoindolyl-, Benzimidazolyl-, Benzofuranyl-, Benzofurazanyl-, Benzothienyl-, Benzthiazolyl-, Benzthiadiazolyl-, Chinolinyl- oder Isochinolinylreste, wobei stickstoffhältige mono- oder bicyclische Heteroarylreste der genannten Art, insbesondere unsubstituierte oder substituierte Pyridylreste, auch als N-Oxide vorliegen können, z.B. Pyridyl-N-oxid.

Ein monocyclischer, carbocyclischer Arylrest $Ar_1$ stellt in erster Linie einen unsubstituierten oder ein- oder mehrfach, z.B. zwei-oder dreifach, substituierten Phenylrest dar, während ein monocyclischer Heteroarylrest $Ar_1$ beispielsweise einen monocyclischen Monooxa-, Monoaza- oder Monothiaarylrest, etwa solche, wie für R definiert, in erster Linie unsubstituiertes oder ein- oder mehrfach, z.B. zwei- oder dreifach, gleich oder verschieden substituiertes Furyl, Pyridyl bzw. Thienyl bedeutet.

Die carbocyclischen und heterocyclischen Arylreste R sowie $Ar_1$ sowie der in einer Alkylengruppe Alk als Substituent enthaltene carbocyclische Rest können unsubstituiert oder ein- oder mehrfach, z.B. zwei- oder dreifach, substituiert sein, wobei in erster Linie Ringkohlenstoffatome, aber auch Ringstickstoffatome substituiert, ein- bis dreifach vorhanden und gleich oder verschieden, vorzugsweise jedoch einfach vorhanden sein können. Substituenten von Ringkohlenstoffatomen sind u.a. gegebenenfalls substituierte Kohlenwasserstoffreste, wie entsprechende aliphatische, cycloaliphatische, aromatische oder araliphatische Kohlenwasserstoffreste, z.B. Niederalkyl, Niederalkenyl, Niederalkinyl, Niederalkylen, Cycloalkyl, Cycloalkylniederalkyl, Phenyl oder Phenylniederalkyl. Substituenten von solchen Kohlenwasserstoffesten, insbesondere von

Niederalkyl, Phenyl oder Phenylniederalkyl, sind z.B. gegebenenfalls verätherte oder veresterte Hydroxygruppen, wie Hydroxy, gegebenenfalls, z.B. durch gegebenenfalls veräthertes oder verestertes Hydroxy, substituiertes Niederalkoxy, z.b. Niederalkoxy, Niederalkoxyniederalkoxy oder Halogenniederalkoxy, gegebenenfalls, z.B. durch gegebenenfalls veräthertes oder verestertes Hydroxy, substituiertes Niederalkenyloxy, z.B. Niederalkenyloxy oder Halogenniederalkenyloxy, Niederalkinyloxy, Niederalkylendioxy, Niederalkanoyloxy oder Halogen, und/oder gegebenenfalls funktionell abgewandeltes Carboxy, wie Carboxy, verestertes Carboxy, z.B. Niederalkoxycarbonyl, amidiertes Carboxy, wie Carbamoyl, N-Niederalkyl-carbamoyl oder N,N-Dinierderalkyl-carbamoyl, oder Cyan. Cyclische Substituenten, insbesondere Phenyl, können zudem auch Niederalkyl, das gegebenenfalls, z.B. wie angegeben, substituiert sein kann, als Substituenten enthalten. Weitere Substituenten von Arylresten R sowie $Ar_1$ ferner das in einer Alkylengruppe Alk enthaltenen carbocyclischen Arylrestes sind z.B. gegebenenfalls verätherte oder veresterte Hydroxygruppen, wie Hydroxy, gegebenenfalls, z.B. durch gegebenenfalls veräthertes oder verestertes Hydroxy, substituiertes Niederalkoxy, z.B. Niederalkoxy, Niederalkoxyniederalkoxy oder Halogenniederalkoxy, gegebenenfalls, z.B. durch gegebenenfalls veräthertes oder verestertes Hydroxy, substituiertes Niederalkenyloxy, z.B. Niederalkenyloxy oder Halogenniederalkenyloxy, Niederalkinyloxy, Niederalkylendioxy, Niederalkanoyloxy oder Halogen, Nitro, gegebenenfalls substituiertes Amino, wie Amino, N-Niederalkylamino, N,N-Diniederalkylamino, N-Niederalkyl-N-phenylniederalkylamino, Niederalkylenamino, Oxaniederalkylenamino, Thianiederalkylenamino oder Azaniederalkylenamino, wobei der Aza-Stickstoff unsubstituiert oder, z.B. durch gegebenenfalls, z.B. wie oben beschrieben, substituiertes Niederalkyl, Phenyl oder Phenylniederalkyl substituiert sein kann, oder Acylamino, z.B. Niederalkylanoylamino, Azido, Acyl, wie Niederalkanoyl oder gegebenenfalls funktionell abgewandeltes Carboxy, wie Carboxy, verestertes Carboxy, z.B. Niederalkoxycarbonyl, oder amidiertes Carboxy, wie Carbamoyl, N-Niederalkyl-carbamoyl oder N,N-Diniederalkyl-carbamoyl, ferner Cyan, gegebenenfalls funktionell abgewandeltes Sulfo, wie Sulfo, Aminosulfonyl, N-Niederalkylaminosulfonyl oder N,N-Diniederalkylaminosulfonyl und/oder veräthertes Mercapto, das gegebenenfalls oxidiert sein kann, wie Niederalkylthio, Halogenniederalkylthio, Niederalkylsulfinyl oder Niederalkylsulfonyl. Substituenten von Ringstickstoffatomen sind in erster Linie die obengenannten, gegebenenfalls substituierten Kohlenwasserstoffreste, wie Niederalkyl, ferner Hydroxy oder Oxido.

Heterocyclische Arylreste R bzw. $Ar_1$ können, z.B. je nach Art der Substitution, in verschiedenartigen tautomeren Formen vorliegen.

Der durch carbocyclisches Aryl substituierte Alkylenrest Alk trennt die Gruppe X vom Ringstickstoffatom durch 2 bis 8 Kohlenstoffatome und ist z.B. entsprechend substituiertes Aethylen, 1,2- oder 1,3-Propylen, 1,4-Butylen oder 1,6-Hexylen, wobei das carbocyclische Aryl an jedem beliebigen Kohlenstoffatom in der unverzweigten Kette, insbesondere aber an dem an die Gruppe X gebundenen Kohlenstoffatom gebunden sein kann. Dementsprechend ist der Alkylenrest Alk beispielsweise 1- oder 2-(Carbocyclisches Aryl)-äthylen, oder 1- oder 2- oder 3-(Carbocyclisches Aryl)-propylen.

Alkylen als Gruppe Y hat 1 bis 6 Kohlenstoffatome und ist z.B. Methylen, Aethylen, 1,2- oder 1,3-Propylen, 1,4-Butylen, 1,5-Pentylen oder 1,6-Hexylen.

Die vor-, sowie nachstehend verwendeten Definitionen haben, sofern nicht besonders definiert, die folgenden Bedeutungen:

Der Ausdruck "nieder" bedeutet, dass entsprechend definierte Gruppen oder Verbindungen, sofern nicht anders definiert, bis und mit 7, vorzugsweise bis und mit 4, Kohlenstoffatome enthalten.

Substituierte Reste können einen oder mehrere, z.B. zwei, ferner drei, gleiche oder verschiedene Substituenten enthalten; diese können irgendeine geeignete Stellung einnehmen.

Naphthyl kann 1- oder 2-Naphthyl sein.

Pyrryl ist z.B. 2- oder 3-Pyrryl, Pyrazolyl z.B. 3- oder 4-Pyrazolyl, Imidazolyl z.B. 2- oder 4-Imidazolyl, Triazolyl z.B. 1,3,5-1H-Triazol-2-yl oder 1,3,4-Triazol-2-yl, und Tetrazolyl z.B. 1,2,3,4-1H-Tetrazolyl-5-yl, während Furyl 2- oder 3-Furyl und Thienyl 2- oder 3-Thienyl bedeuten. Isoxazolyl ist z.B. 3-Isoxazolyl, Oxazolyl z.B. 2- oder 4-Oxazolyl, Oxadiazolyl z.B. 1,3,4-Oxadiazol-2-yl, Isothiazolyl z.B. 3-Isothiazolyl, Thiazolyl 2- oder 4-Thiazolyl, und Thiadiazolyl z.B. 1,3,4-Thiadiazolyl-2-yl.

Pyridyl ist 2-, 3- oder 4-Pyridyl, Pyridazinyl z.B. 3-Pyridazinyl, Pyrimidinyl 2-, 4- pder 5-Pyrimidinyl, Pyrazinyl, 2-Pyrazinyl und Triazinyl z.B. 1,3,5-Triazin-2-yl.

Indolyl ist z.B. 2-, 3- oder 5-Indolyl, Isoindolyl z.B. 1-Isoindolyl, Benzimidazolyl z.B. 2- oder 5-Benzimidazolyl, Benzofuranyl z.B. 2- oder 3-Benzofuranyl, Benzofuranyl (auch 2,1,3-Benzoxadiazolyl) z.B. 4-Benzofurazanyl, Benzothienyl z.B. 3-Benzothienyl, Benzthiazolyl z.B. 2-Benzthiazolyl, 2,1,3-Benzthiadiazolyl z.B. 2,1,3-Benzthiadiazol-4-yl, Chinolinyl z.B. 2- oder 4-Chinolinyl, und Isochinolinyl z.B. 1-Isochinolinyl.

1-Oxido-Pyridyl ist z.B. 1-Oxido-2-, 1-Oxido-3- oder 1-Oxido-4-pyridyl.

Niederalkyl ist z.B. Methyl, Aethyl, n-Propyl, Isopropyl, n-Butyl, Isobutyl oder tert.-Butyl, ferner n-Pentyl, n-Hexyl oder n-Heptyl, während Niederalkenyl z.B. Allyl oder Methallyl, und Niederalkinyl z.B. Propargyl bedeutet, wobei die Mehrfachbindung jeweils bevorzugt in höherer als der $\alpha$-Stellung lokalisiert ist.

Cycloalkyl weist vorzugsweise 5 bis 7 Ringkohlenstoffatome auf und ist z.B. Cyclopentyl oder Cyclohexyl, während Cycloalkylniederalkyl z.B. Cyclopropylmethyl, Cyclopentylmethyl oder Cyclohexylmethyl sein kann.

Phenylniederalkyl ist z.B. Benzyl oder 1- oder 2-Phenyläthyl.

Niederalkoxy steht in erster Linie für Methoxy, Aethoxy, n-Propyloxy, Isopropyloxy, n-Butyloxy, Isobutyloxy oder tert.-Butyloxy.

In einem Niederalkoxyniederalkoxyrest ist die terminale Niederalkoxygruppe vorzugsweise durch mehr als

ein Kohlenstoffatom vom Verknüpfungssauerstoffatom getrennt; solche Reste sind z.B. 2-Methoxyäthoxy oder 2-Aethoxy-äthoxy.

In einem Halogen-niederalkoxyrest können ein oder mehrere Halogenatome, die vorzugsweise eine Atomnummer bis 35 aufweisen und besonders für Fluor und/oder Chlor stehen, vorhanden sein; solche Reste sind z.B. Difluormethoxy, 1,1,2,2-Tetrafluoräthoxy oder 1,1,2-Trifluor-2-chlor-äthoxy.

Niederalkenyloxy ist z.B. Allyloxy oder Methallyloxy, und Halogenniederalkenyloxy, das ein oder mehrere Halogenatome enthalten kann, wobei letztere vorzugsweise eine Atomnummer bis 35 aufweisen und besonders für Fluor und/oder Chlor stehen, ist z.B. 1,2-Dichlorvinyloxy, wobei die Doppelbindung jedoch bevorzugt in höherer als der $\alpha$-Stellung lokalisiert ist.

Niederalkinyloxy ist z.B. Propargyloxy, wobei die Dreifachbindung bevorzugt in höherer als der $\alpha$-Stellung lokalisiert ist.

Niederalkylen ist z.B. 1,2-Aethylen oder 1,3-Propylen, während Niederalkylendioxy z.B. Methylendioxy oder 1,2-Aethylendioxy ist.

Niederalkanoyloxy ist z.B. Acetyloxy, Propionyloxy oder Pivaloyloxy.

Halogen hat vorzugsweise eine Atomnummer bis und mit 35 und ist insbesondere Fluor oder Chlor, ferner Brom, kann jedoch auch Jod sein.

Halogensubstituiertes Niederalkyl ist z.B. Trifluormethyl, 1,1,2-Trifluor-2-chlor-äthyl, 1,1,2,2-Tetrafluoräthyl oder Chlormethyl.

Niederalkoxycarbonyl ist z.B. Methoxycarbonyl, Aethoxycarbonyl, n-Propyloxycarbonyl, Isopropyloxycarbonyl, n-Butyloxycarbonyl, Isobutyloxycarbonyl oder tert.-Butyloxycarbonyl.

N-Niederalkyl-carbamoyl ist z.B. N-Methyl-carbamoyl oder N-Aethylcarbamoyl, während N,N-Diniederalkylcarbamoyl z.B. N,N-Dimethyl-carbamoyl oder N,N-Diäthyl-carbamoyl ist.

N-Niederalkylamino ist z.B. N-Methylamino, N-Aethylamino, N-n-Propylamino oder N-Isopropylamino.

N,N-Diniederalkylamino ist z.B. N,N-Dimethylamino, N-Aethyl-N-methylamino oder N,N-Diäthylamino, während N-Niederalkyl-N-phenylniederalkylamino z.B. N-Benzyl-N-methylamino oder N-Methyl-N-(2-phenyläthyl)-amino darstellt.

Niederalkylenamino enthält vorzugsweise 4 bis 6 Ringkohlenstoffatome und ist z.B. Pyrrolidino oder Piperidino, während Oxaniederalkylenamino z.B. 4-Morpholino, Thianiederalkylenamino z.B. 4-Thiomorpholino, und gegebenenfalls azasubstituiertes Azaniederalkylenamino z.B. Piperazino, 4-Methylpiperazino, 4-Phenyl-piperazino, 4-Benzyl-piperazino oder 4-(2-Phenyläthyl)-piperazino darstellen kann.

Niederalkylanoylamino ist z.B. Acetylamino oder Propionylamino.

Niederalkanoyl ist z.B. Formyl, Acetyl, Propionyl oder Pivaloyl.

Niederalkylthio ist z.B. Methylthio, Aethylthio, n-Propylthio oder Isopropylthio, während Halogenniederalkylthio insbesondere Fluorniederalkylthio, z.B. Mono- oder Difluormethylthio darstellt. Niederalkylsulfinyl ist z.B. Methylsulfinyl, während Niederalkylsulfonyl z.B. Methylsulfonyl oder Aethylsulfonyl bedeutet.

N-Niederalkylaminosulfonyl ist z.B. N-Methylaminosulfonyl, während N,N-Diniederalkylaminosulfonyl z.B. N,N-Dimethylaminosulfonyl ist.

In einem substituierten Niederalkoxycarbonyl ist der Substituent üblicherweise durch mindestens 2, vorzugsweise durch 2 oder 3 Kohlenstoffatome vom Sauerstoff getrennt. Solche Reste sind z.B. Hydroxyniederalkoxycarbonyl, wie 2-Hydroxyäthoxycarbonyl oder 2,3-Dihydroxypropylcarbonyl, Niederalkoxyniederalkoxycarbonyl, z.B. 2-Methoxyäthoxycarbonyl, diniederalkylamio-niederalkoxycarbonyl, z.B. 2-Dimethylaminoäthoxycarbonyl, 2-Diäthylaminoäthoxycarbonyl oder 3-Dimethylaminopropyloxycarbonyl, Niederalkylenaminoniederalkoxycarbonyl, z.B. 2-Pyrrolidinoäthoxycarbonyl oder 2-Piperidinoäthoxycarbonyl, Morpholino-niederalkoxycarbonyl, z.B. 2-(4-Morpholino)-äthoxycarbonyl, oder (4-Niederalkyl-piperazino)-n-niederalkoxycarbonyl, z.B. 2-(4-Methylpiperazino)-äthoxycarbonyl.

Phenylniederalkoxycarbonyl ist z.B. Benzyloxycarbonyl oder 2-Phenyläthoxy-carbonyl.

N,N-Niederalkylen-carbamoyl ist z.B. Pyrrolidinocarbonyl oder Piperidinocarbonyl, wobei entsprechende Reste, in welchen der Niederalkylenteil durch Sauerstoff, Schwefel oder unsubstituierten oder substituierten Stickstoff unterbrochen ist, z.B. 4-Morpholinocarbonyl, 4-Thiomorpholinocarbonyl, 1-Piperazinocarbonyl oder 4-Methyl-1-piperazinocarbonyl bedeutet.

Verbindungen der Formel I können in Form von Salzen, insbesondere von Säureadditionssalzen, in erster Linie entsprechenden pharmazeutisch verwendbaren, nicht-toxischen Säureadditionssalzen, vorliegen. Solche Salze sind z.B. diejenigen mit Halogenwasserstoffsäuren, z.B. Chlorwasserstoffsäure oder Bromwasserstoffsäure, ferner Salpetersäure, Schwefelsäure oder Phosphorsäure, oder organischen Säuren, wie Carbonsäuren, z.B. Essigsäure, Propionsäure, Glykolsäure, Oxalsäure, Bernsteinsäure, Maleinsäure, Hydroxymaleinsäure, Methylmaleinsäure, Fumarsäure, Aepfelsäure, Weinsäure, Zitronensäure, Benzoesäure, Zimtsäure, Mandelsäure, Salicylsäure, 4-Amino-salicylsäure, 2-Phenoxybenzoesäure, 2-Acetoxybenzoesäure, Embonsäure, Nicotinsäure oder Isonicotinsäure, ferner Aminosäuren, oder organischen Sulfonsäuren, wie gegebenenfalls Hydroxy enthaltende Niederalkansulfonsäuren, z.B. Methansulfonsäure, Aethansulfonsäure, 2-Hydroxyäthansulfonsäure oder Aethan-1,2-disulfonsäure, oder Arylsulfonsäure, z.B. Benzolsulfonsäure, 4-Methylbenzolsulfonsäure oder Naphthalin-2-sulfonsäure, oder mit anderen sauren organischen Substanzen, wie Ascorbinsäure.

Umfasst sind ferner für pharmazeutische Verwendung ungeeignete Salze, da diese beispielsweise für die Isolierung bzw. Reinigung freier erfindungsgemässer oder deren pharmazeutisch verwendbaren Salze verwendet werden können.

Die Verbindungen der Formel I und deren Salze besitzen wertvolle pharmakologische Eigenschaften, vor allem im cardiovasculären Bereich. Sie wirken als Calcium-Antagonisten und besitzen α-Rezeptoren blockierende sowie Serotonin-antagonistische Eigenschaften, wie sich z.B. in in-vitro-Versuchen an isoliert perfundierten Mesenterialgefässen der Ratte [McGregor, D.: J.Physiol. 177, 21-30, (1965)] als Hemmung der Kalium-,Noradrenalin-und Serotonin-induzierten Vasokonstriktion, beispielsweise unter Verwendung von 2,6-Dimethyl-4-(3-nitrophenyl)-1,4-dihydropyridin-3-carbonsäurmethylester-5-carbonsäure-[2-[4-(p-fluorbenzoyl)-piperidin-1-yl] -1-phenyl]-äthylester (Diastereomerengemisch) bei einer Konzentration von ca. 5 nMol/Liter ($IC_{50}$) für die Hemmung der Kalium-induzierten Vasokonstriktion, von ca. 4 nMol/Liter ($IC_{50}$) für die Hemmung der Noradrenalin-induzierten Vasokonstriktion und von ca. 80 nMol/Liter für die Hemmung der Serotonin-induzierten Vasokonstriktion zeigen lässt.

Ferner lässt sich die Bindung der neuen Verbindungen oder deren Salze an Dihydropyridin-Rezeptoren mittels Verdrängung der Bindung von $^3$H-Nitrendipin an Membranen von Meerschweichenherzen in in-vitro-Versuchen [Erne, P. et al,: Biochem. Biophys. Res. Commm., 118, 842-847 (1984)] z.B. unter Verwendung von 2,6-Dimethyl-4-(3-nitrophenyl)-1,4-dihydropyridin-3-carbonsäuremethylester-5-carbonsäure-[2-[4-(p-fluorobenzoyl)-piperidin-1-yl]-1-phenyl] -äthyl ester (Diastereomerengemisch) bei einer Konzentration ($IC_{50}$) von etwa 0,2 nMol/Liter nachweisen. Ebenso kann die Bindung der erfindungsgemässen Verbindungen an $\alpha_1$-Rezeptoren durch Verdrängung von $^3$H-Prazosin aus seinen Bindungsstellen an der Ratten-Cerebralcortex-Membran gemäss der Methodik von P. Greenengass et al., Eur. J. Pharmacol. 55, 323, (1979), bei etwa 300 nMol/Liter in vitro signifikant nachgewiesen werden. Ausserdem lässt sich die Bindung der neuen Verbindungen oder deren Salze an Serotonin-($5HT_2$)-Rezeptoren mittels Verdrängung der Bindung von $^3$H-Ketanserin an Membranen des Rattenhirns [Leysen, J.E. et al.: Molecul. Pharmacol. 21, 301-304, (1982)] z.B. unter Verwendung von 2,6-Dimethyl-4-(3-nitrophenyl)-1,4-dihydropyridin-3-carbonsäuremethylester-5-carbonsäure-[2-[4-(p-fluorbenzoyl)-piperidin-1-yl]-1-phenyl]-äthylester (Diastereomerengemisch) bei einer Konzentration ($IC_{50}$) von etwa 6,0 nMol/Liter zeigen.

Die neuen Verbindungen oder deren Salze bewirken ferner eine Hemmung der durch Angiotensin II, durch elektrische Stimulation oder durch Phenylephrin oder Noradrenalin induzierten pressorischen Effekte an der "pithed rat" [J.S. Gillespie: Br.J.Pharmacol. 30, 78 (1967)] bei einer Dosis von etwa 2 mg/kg, und eine Hemmung der durch Serotonin induzierten pressorischen Effekte bei einer Dosis von etwa 6 mg/kg. Die neuen Verbindungen oder deren Salze wirken ausserdem antihypertensiv, was sich z.B. durch Senkung des Blutdrucks an der renal hypertensiven Ratte [Goldblatt et al.: J.exp.med. 59, 347 (1934)] zeigen lässt. So kann z.B. 2 Stunden nach p.o-Applikation von ca. 2,0 mg/kg 2,6-Dimethyl-4-(3-nitrophenyl)-1,4-dihydropyridin-3-carbonsäuremethylester-5-carbonsäure-[2-[4-(p-fluorbenzoyl)-piperidin-1-yl]-1-phenyl]-äthylester (Diastereomerengemisch) an der renal hypertensiven Ratte eine Blutdrucksenkung von ca. -70 mm Hg beobachtet werden.

Die neuen Verbindungen und deren Salze weisen ausserdem als Besonderheit eine Affinität zu Bindungsstellen des Calciumkanals und zu $\alpha_1$- und $5HT_2$-Rezeptoren auf und unterscheiden sich dadurch von anderen Calcium-Antagonisten.

Die Verbindungen der Formel I und Salze von solchen Verbindungen können daher z.B. als Coronardilatatoren und Antihypertensiva zur Behandlung von cardiovasculären Krankheitszuständen, wie Angina pectoris und ihren Folgen, zentralen und peripheren Durchblutungsstörungen, hohem Blutdruck, Arrhythmien und Herzinsuffizienz, Verwendung finden. Die neuen Verbindungen sind aber auch wertvolle Zwischenprodukte zur Herstellung anderer, insbesondere pharmazeutisch wirksamer Verbindungen. Ein weitere Gegenstand der Erfindung ist die Verwendung der erfindungsgemässen Verbindungen zur Herstellung von Arzneimitteln, z.B. zur Behandlung von cardiovasculären Krankheitszuständen und Bluthochdruck, zur therapeutischen und prophylaktischen Behandlung. Dabei kann auch die gewerbsmässige Herrichtung der Wirksubstanzen eingeschlossen sein.

Die Erfindung betrifft insbesondere neue Verbindungen der Formel I in welchen R für einen mono- oder bicyclischen, carbocyclischen Arylrest oder für einen fünf- oder sechsgliedrigen, ein bis und mit vier Ringstickstoffatome, ein Ringsäuerstoff- oder Ringschwefelatom, oder ein oder zwei Ringstickstoffatome zusammen mit einem Ringsauerstoff- oder einem Ringschwefelatom als Ringglieder enthaltenden, monocyclischen Heteroarylrest steht, der über ein Ringkohlenstoffatom mit dem Kohlenstoffatom der 4-Stellung des 1,4-Dihydropyridinrings verbunden ist, und der gegebenenfalls einen ankondensierten Benzoring enthält, und insbesondere Phenyl, Naphthyl, Pyrryl, Pyrazolyl, Imidazolyl, Triazolyl, Tetrazolyl, Furyl, Thienyl, Isoxazolyl, Oxazolyl, Oxadiazolyl, Isothiazolyl, Thiazolyl, Thiadiazolyl, Pyridyl, Pyridazinyl, Pyrimidinyl, Pyrazinyl, Triazinyl, Indolyl, Isoindolyl, Benzimidazolyl, Benzofuranyl, Benzofurazanyl, Benzothienyl, Benzthiazolyl, 2,1,3-Benzthiadiazolyl, Chinolinyl oder Isochinolinyl bedeutet, wobei in diesen Resten Ringkohlenstoffatome gegebenenfalls durch Niederalkyl, Niederalkenyl, Niederalkinyl, Niederalkylen, Cycloalkyl, Phenyl und/oder Phenylniederalkyl, wobei Niederalkyl, Phenyl oder Phenylniederalkyl gegebenenfalls Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkylcarbamoyl und/oder Cyan, und cyclische Reste auch Niederalkyl, das seinerseits wie angegeben substituiert sein kann, als Substituenten enthalten können, und/oder durch Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Nitro, Amino, N-Niederalkyl-amino, N,N,-Diniederalkylamino, N-Niederalkyl-N-phenylniederalkyl-amino, Niederalkylenamino,

Oxaniederalkylenamino, Thianiederalkylenamino und/oder Azaniederalkylenamino, worin das Azastickstoffatom durch Niederalkyl, Phenyl oder Phenylniederalkyl substituiert sein kann, wobei diese Reste Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkyl-carbamoyl und/oder Cyan, die cyclischen Reste auch Niederalkyl als Substituenten enthalten können und/oder durch Niederalkanoylamino, Azido, Niederalkanoyl, Carboxy, Niederalkoxycarbonyl, Carbamoyl, N-Niederalkyl-carbamoyl, N,N-Diniederalkyl-carbamoyl, Cyan, Sulfo, Aminosulfonyl, N-Niederalkylaminosulfonyl, N,N-Diniederalkylaminosulfonyl, Niederalkylthio, Halogenniederalkylthio, Niederalkylsulfinyl und/oder Niederalkylsulfonyl, und/oder Ringstickstoffatome gegebenenfalls durch Niederalkyl, das als Substituenten gegebenenfalls Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkyl-carbamoyl oder Cyan enthalten kann, oder durch Hydroxy oder Oxido substituiert sein können, $R_1$ Niederalkyl ist, einer der Reste $R_2$ und $R_3$ für Niederalkyl und der andere für Niederalkyl, Cyano oder Amino steht, X Sauerstoff oder die Gruppe -NH- bedeutet, und Alk durch ein carbocyclisches Aryl, z.B. durch gegebenenfalls, etwa wie nachfolgend für carbocyclisches Aryl $Ar_1$ angegeben, substituiertes Phenyl oder Naphthyl, substituiertes Alkylen darstellt, das die Gruppe X vom Ringstickstoffatom durch 2 bis 8 Kohlenstoffatome trennt, Y für Alkylen mit 1 bis 6 Kohlenstoffatomen oder für die Gruppen -CH(OH)- oder -C(=O)- steht oder eine einfache Bindung bedeutet, und $Ar_1$ einen monocyclischen carbocyclischen Aryl- oder Heteroarylrest darstellt, und insbesondere Phenyl, Naphthyl, Pyrryl, Furyl, Thienyl oder Pyridyl bedeutet, wobei in diesen Resten Ringkohlenstoffatome gegebenenfalls durch Niederalkyl, Niederalkenyl, Niederalkinyl, Niederalkylen, Cycloalkyl, Phenyl und/oder Phenylniederalkyl substituiert sind, wobei Niederalkyl, Phenyl oder Phenylniederalkyl als Substituenten Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkyl-carbamoyl und/oder Cyan, und cyclische Reste auch Niederalkyl, das seinerseits wie angegeben substituiert sein kann, enthalten können, und/oder durch Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Nitro, Amino, N-Niederalkyl-amino, N,N-Diniederalkylamino, N-Niederalkyl-N-phenylniederalkyl-amino, Niederalkylenamino, Oxoniederalkylenamino, Thianiederalkylenamino und/oder Azaniederalkylenamino, worin das Azastickstoffatom durch Niederalkyl, Phenyl oder Phenylniederalkyl substituiert sein kann, wobei diese Reste Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkyl-carbamoyl und/oder Cyan, die cyclischen Reste auch Niederalkyl als Substituenten enthalten können, und/oder durch Niederalkylanoylamino, Azido, Niederalkanoyl, Carboxy, Niederalkoxycarbonyl, Carbamoyl, N-Niederalkyl-carbamoyl, N,N-Diniederalkyl-carbamoyl, Cyan, Sulfo, Aminosulfonyl, N-Niederalkylaminosulfonyl, N,N-Diniederalkylaminosulfonyl, Niederalkylthio, Niederalkylsulfinyl und/oder Niederalkylsulfonyl, und/oder Ringstickstoffatome gegebenenfalls durch Niederalkyl, das als Substituenten gegebenenfalls Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkylcarbamoyl, N,N-Diniederalkyl-carbamoyl oder Cyan als Substituenten enthalten kann, oder durch Hydroxy oder Oxido substituiert sein können, sowie Salze, insbesondere pharmazeutisch verwendbare, nichttoxische Säureadditionssalze solcher Verbindungen.

Die Erfindung betrifft in erster Linie neue Verbindungen der Formel I, worin R und $Ar_1$ jeweils für Phenyl oder Naphthyl stehen, das gegebenenfalls durch Niederalkyl, Phenyl und/oder Phenylniederalkyl, wobei solche Reste ihrerseits Hydroxy, Niederalkoxy, Halogenniederalkoxy, Niederalkylendioxy, Halogen, Carboxy, Niederalkoxycarbonyl und/oder Cyan, die cyclischen Reste auch Niederalkyl als Substituenten enthalten können, und/oder durch Hydroxy, Niederalkoxy, Halogenniederalkoxy, Niederalkenyloxy, Halogenniederalkenyloxy, Niederalkylendioxy, Halogen, Nitro, Amino, N-Niederalkylamino, N,N-Diniederalkylamino, Niederalkanoylamino, Carboxy, Niederalkoxycarbonyl, Carbamoyl, Cyan, Sulfo, Aminosulfonyl, N-Niederalkylaminosulfonyl, N,N-Diniederalkylaminosulfonyl, Niederalkylthio, Niederalkylsulfinyl und/oder Niederalkylsulfonyl substituiert ist, oder R und $Ar_1$ jeweils für über ein Ringkohlenstoffatom gebundenes Pyrryl, Furyl, Thienyl, Pyridyl, 1-Oxido-pyridyl oder Imidazolyl, R auch für Benzofurazanyl steht, wobei solche Reste gegebenenfalls, wie für einen Phenyl- oder Naphthylrest R bzw. $Ar_1$ angegeben, substituiert sind, und insbesondere Niederalkyl, Niederalkoxy, Halogen und/oder gegebenenfalls durch Niederalkyl, Niederalkoxy, Halogen und/oder Nitro substituiertes Phenyl als Substituenten enthalten können, $R_1$ Niederalkyl ist, eine der Gruppen $R_2$ und $R_3$ Niederalkyl ist und die andere Niederalkyl oder Cyano darstellt, X Sauerstoff oder die Gruppe -NH- bedeutet, und Alk durch ein gegebenenfalls durch Niederalkyl, Niederalkoxy, Hydroxy, Halogenniederalkyl, Halogen, Cyan und/oder Nitro substituierte Phenyl oder Naphthyl substituiertes Alkylen mit 2 bis 6 Kohlenstoffatomen steht, das die Gruppe X vom Ringstickstoffatom durch 2 bis 6 Kohlenstoffatome trennt und Y Alkylen mit 1 bis 4 Kohlenstoffatomen oder die Gruppen -CH(OH)- oder -C(=O) bedeutet, sowie Salze, insbesondere pharmazeutisch verwendbare, nicht-toxische Säureadditionssalze solcher Verbindungen.

Die Erfindung betrifft ganz besonders neue Verbindungen der Formel I, worin R und $Ar_1$ jeweils für Phenyl stehen, das gegebenenfalls durch Niederalkyl, Hydroxy, Niederalkoxy, Halogenniederalkoxy, Halogenniederalkenyloxy, Niederalkylendioxy, Halogen, Trifluormethyl, Nitro, Niederalkanoylamino, gegebenenfalls durch gegebenenfalls verestertes oder veräthertes Hydroxy substituiertes Phenyl oder Phenylniederalkyl, und/oder Cyan substituiert ist, wobei ein Phenylrest R bzw. $Ar_1$ einen oder mehrere, gleiche oder verschiedene Substituenten aufweisen kann, oder R und $Ar_1$ jeweils Pyridyl, Furyl, 1-Oxido-pyridyl, oder Thienyl, R auch Benzofuranyl, z.B. 4-Benzofurazanyl, bedeutet, das durch Niederalkyl oder Halogen substituiert sein kann, $R_1$, $R_2$ und $R_3$ jeweils Niederalkyl bedeuten, X für die Gruppe -NH-, insbesondere jedoch für Sauerstoff steht, Alk durch ein gegebenenfalls Niederalkyl, Niederalkoxy, Hydroxy, Halogenniederalkyl, Halogen, Cyan und/oder Nitro enthaltendes Phenyl substituiertes Alkylen mit 2 bis 4 Kohlenstoffatomen bedeutet, wobei der gegebenenfalls substituierte Phenylrest vorzugsweise an das an die Gruppe X benachbarte Kohlenstoffatom gebunden ist, und Alk die Gruppe X vom Ringstickstoffatom durch 2 bis 4 Kohlenstoffatome trennt, Y für Alkylen mit 1 bis 3 Kohlenstoffatomen oder die Gruppen -CH(OH)- oder insbesondere -C(=O)- steht, sowie Salze, insbesondere pharmazeutisch verwendbare nicht-toxische Säureadditionssalze solcher Verbindungen.

Die Erfindung betrifft insbesondere neue Verbindungen der Formel I, worin R für unsubstituiertes oder vorzugsweise durch Niederalkyl, z.B. Methyl, Niederalkoxy, z.B. Methoxy, Halogenniederalkoxy, z.B. Difluormethoxy, oder Halogenniederalkenyloxy, worin Halogen eine Atomnummer bis und mit 35 hat und insbesondere Fluor oder Chlor ist, z.B. 1,1-Difluorvinyloxy, Halogen mit einer Atomnummer bis und mit 35, insbesondere Chlor sowie Fluor, Trifluormethyl, Nitro und/oder Cyan mono- oder di-substituiertes Phenyl steht, wobei Substituenten die 2- und/oder 3-Stellung einnehmen, $R_1$ Niederalkyl z.B.Methyl oder Aethyl, und $R_2$ und $R_3$ Niederalkyl, insbesondere Methyl, bedeuten, X für die Gruppe -NH-, insbesondere jedoch für Sauerstoff steht, Alk durch ein durch Niederalkyl, Niederalkoxy, Hydroxy, Halogenniederalkyl, Halogen, Cyan und/oder Nitro substituiertes, insbesondere aber durch ein unsubstituiertes Phenyl substituiertes Alkylen mit 1 bis 3 Kohlenstoffatomen darstellt, wobei der gegebenenfalls substituierte Phenylrest an das der Gruppe X benachbarte Kohlenstoffatome gebunden ist, und Alk die Gruppe X vom Ringstickstoffatom durch 2 bis 3 Kohlenstoffatome trennt, Y für Alkylen mit 1 bis 3 Kohlenstoffatomen oder insbesondere die Gruppe -C(=O)- steht, und $Ar_1$ gegebenenfalls durch Halogen mit einer Atomnummer bis und mit 35, z.B. Fluor oder Chlor, substituiertes Phenyl oder Pyridyl bedeutet, sowie Salze, insbesondere pharmazeutisch verwendbare, nicht-toxische Säureadditionssalze solcher Verbindungen.

Die Erfindung betrifft insbesondere neue Verbindungen der Formel I, worin R durch Halogenniederalkoxy, insbesondere mit einer Atomnummer bis und mit 35 und mit bis 4 C-Atomen, wie Difluormethoxy, Niederalkylendioxy, insbesondere mit bis und mit 3 C-Atomen, wie Methylendioxy, Halogen, insbesondere mit einer Atomnummer bis und mit 35, wie Chlor, Trifluomethyl oder Nitro mono- oder disubstituiertes Phenyl, wobei Substituenten die 2- und/oder 3-Stellung einnehmen, Benzofurazanyl, insbesondere 4-Benzofurazanyl, oder 1-Oxido-pyridyl, wie 1-Oxido-3-pyridyl, bedeutet, $R_1$, $R_2$ und $R_3$ unabhängig voneinander Niederalkyl, insbesondere mit bis und mit 4 C-Atomen, wie Methyl, bedeuten, X für Sauerstoff steht, Alk durch ein gegebenenfalls Niederalkyl, Niederalkoxy, jeweils insbesondere mit bis und mit 4 C-Atomen, wie Methyl oder Methoxy, Halogenniederalkyl, insbesondere mit einer Atomnummer bis und mit 35 und mit bis und mit 4 C-Atomen, wie Trifluormethyl, Halogen, insbesondere mit einer Atomnummer bis und mit 35, wie Chlor, oder Cyan substituiertes Phenyl enthaltendes Alkylen mit 2 bis 4 C-Atomen bedeutet, wobei Alk die Gruppe X vom Ringstickstoffatom durch 2 bis 4 C-Atome trennt, Y für Alkylen mit 1 bis 3 C-Atomen, die Gruppen -CH(OH)- oder insbesondere -C(=O)- oder eine Bindung steht, und $Ar_1$ gegebenenfalls durch Halogen, insbesondere mit einer Atomnummer bis und mit 35, wie Chlor, substituiertes Phenyl oder Thienyl, wie 2-Thienyl, bedeutet, sowie Salze, insbesondere pharmazeutisch verwendbare, nicht-toxische Säureadditionssalze solcher Verbindungen.

Die Erfindung betrifft insbesondere neue Verbindungen der Formel I, worin R durch Halogen mit einer Atomnummer bis und mit 35, wie Chlor, Trifluormethyl oder Nitro mono- oder disubstituiertes Phenyl steht, wobei Substituenten bevorzugt die 2- oder 3-Stellung einnehmen, $R_1$, $R_2$ und $R_3$ unabhängig voneinander Niederalkyl mit bis und mit 4 C-Atomen, wie Methyl oder Aethyl, bedeuten, X für Sauerstoff steht, Alk durch ein durch Niederalkyl, Niederalkoxy jeweils mit bis und mit 4 C-Atomen, wie Methyl oder Methoxy, Halogenniederalkyl mit einer Atomnummer bis und mit 35 und mit bis und mit 4 C-Atomen, wie Trifluormethyl, Halogen mit einer Atomnummer bis und mit 35, wie Chlor, oder Cyan substituiertes oder unsubstituiertes Phenyl substituiertes Alkylen mit 2 bis und mit 6 C-Atomen, bedeutet, wobei der gegebenenfalls substituiertes Phenylrest vorzugsweise an das an die Gruppe X benachbarte C-Atom gebunden ist und die Gruppe X vom Ringstickstoffatom durch mindestens zwei C-Atome getrennt wird, Y für Alkylen mit 1 bis 3 C-Atomen, die Gruppen -CH(OH)- oder -C(=O), ferner eine Bindung steht, und $Ar_1$ unsubstituiertes oder durch Halogen mit einer Atomnummer bis und mit 35, wie Fluor, substituiertes Phenyl oder Thienyl, wie 2-Thienyl, bedeutet, sowie Salze, insbesondere pharmazeutisch verwendbare, nicht-toxische Säureadditionssalze solcher Verbindungen.

Die Erfindung betrifft insbesondere neue Verbindungen der Formel I. worin R für 2-, 3-Nitrophenyl, 2-, 3-Trifluormethylphenyl oder 2,3-Dichlorphenyl steht, $R_1$, $R_2$ und $R_3$ unabhängig voneinander jeweils Niederalkyl mit bis und mit 4 C-Atomen, insbesondere Methyl, bedeuten, X für Sauerstoff steht, Alk durch ein durch Niederalkyl mit bis und mit 4 C-Atomen, wie Methyl, Niederalkoxy mit bis und mit 4 C-Atomen, wie Methoxy, Halogenniederalkyl mit einer Atomnummer bis und mit 35 und mit bis und mit 4 C-Atomen, wie Trifluormethyl, Halogen mit einer Atomnummer bis und mit 35, wie Chlor, oder Cyano monosubstituiertes oder unsubstituiertes Phenyl substituiertes Alkylen mit 2 bis und mit 6 C-Atomen bedeutet und wobei die Gruppe X vom Ringstickstoffatom durch mindestens zwei C-Atome getrennt wird, Y für Methylen, Hydroxymethylen, die

Gruppe -C(=O)- oder eine Bindung steht. und $Ar_1$ unsubstituiertes oder durch Halogen mit einer Atomnummer bis und mit 35, wie Fluor, monosubstituiertes Phenyl oder Thienyl, wie 2-Thienyl, bedeutet, sowie Salze, insbesondere pharmazeutisch verwendbare, nicht-toxische Säureadditionssalze solcher Verbindungen.

Die Erfindung betrifft insbesondere neue Verbindungen der Formel I, worin R für durch Halogen mit einer Atomnummer bis und mit 35, besonders Fluor oder Chlor, mono- oder disubstituiertes oder durch Trifluormethyl, Nitro oder Cyan monosubstituiertes Phenyl steht, wobei Substituenten die 2- und/oder 3-Stellung einnehmen, $R_1$ Niederalkyl, z.B. Methyl oder Aethyl, und $R_2$ und $R_3$ jeweils Niederalkyl, in erster Linie Methyl, bedeuten, X für Sauerstoff steht. Alk durch ein durch Niederalkyl, z.B. Methyl, Niederalkoxy, z.B. Methoxy, Trifluormethyl, Cyan und/oder Nitro substituiertes, insbesondere aber durch ein unsubstituiertes, Phenyl substituiertes Alkylen mit 2 bis 3 Kohlenstoffatomen darstellt, wobei der gegebenenfalls substituierte Phenylrest an das der Gruppe X benachbarte Kohlenstoffatom gebundet ist, und Alk die Gruppe X vom Ringstickstoffatom durch 2 bis 3 Kohlenstoffatome trennt, Y für Alkylen mit 1 bis 2 Kohlenstoffatomen oder insbesondere die Gruppe -C(=O)- steht, und $Ar_1$ gegebenenfalls, insbesondere in 4-Stellung, durch Halogen mit einer Atomnummer bis und mit 35, z.B. Fluor, substituiertes Phenyl bedeutet, sowie Salze, insbesondere pharmazeutisch verwendbare, nicht-toxische Säureadditionssalze solcher Verbindungen.

Die Erfindung betrifft in erster Linie neue Verbindunger der Formel I, worin R 3-Nitrophenyl oder 2-Trifluormethylphenyl bedeutet, $R_1$ Methyl oder Aethyl bedeutet, $R_2$ und $R_3$ Methyl bedeuten, X für Sauerstoff steht, Alk 1-Phenyläthyl bedeutet, Y für die Gruppe -C(=O)- steht und $Ar_1$ gegebenenfalls in 4-Stellung durch Fluor substituiertes Phenyl bedeutet, sowie Salze, insbesondere pharmazeutisch verwendbare, nicht-toxische Säureadditionssalze von solchen Verbindungen.

Die Erfindung betrifft insbesondere die in den Beispielen beschriebenen spezifischen neuen Verbindungen und Verfahren zu ihrer Herstellung.

Die Verbindungen der Formel I und Salze von solchen Verbindungen mit salzbildenden Eigenschaften können in an sich bekannter Weise hergestellt werden, indem man z.B.

a) eine Verbindung der Formel

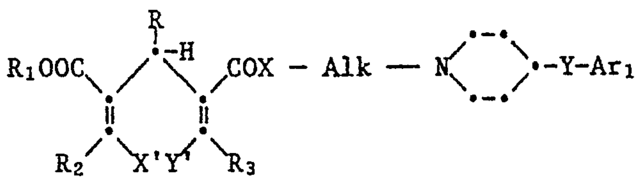

(II),

worin einer der Reste X' und Y' für die Gruppe der Formel $-NH_2$ steht und der andere Hydroxy oder die Gruppe der Formel $-NH_2$ bedeutet, oder ein Tautomeres davon oder ein entsprechendes Tautomerengemisch ringschliesst, oder

b) eine Verbindung der Formel R-CHO (III) oder ein reaktionsfähiges funktionelles Derivat davon mit einer Verbindung der Formel

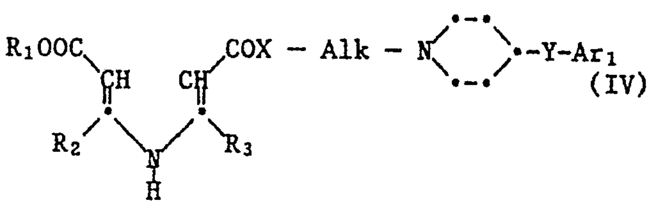

(IV)

oder einem Tautomeren davon oder einem entsprechenden Tautomerengemisch umsetzt, oder

c) in einer Verbindung der Formel

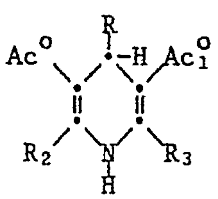

(V),

in welcher einer der Reste $Ac^o$ und $Ac_1^o$ eine in die Gruppe $-COOR_1$ bzw. in den Rest der Formel

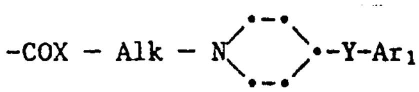

(Va)

überführbare Gruppe bedeutet, und der andere die Gruppe $-COOR_1$ oder die Gruppe der Formel Va oder einen in die Gruppe $-COOR_1$ bzw. die Gruppe der Formel Va überführbaren Rest bedeutet, den Rest $Ac^{o}$ in die Gruppe $-COOR_1$ und/oder den Rest $Ac^{o}$ in einen Rest der Formel Va überführt, oder

d) eine Verbindung der Formel

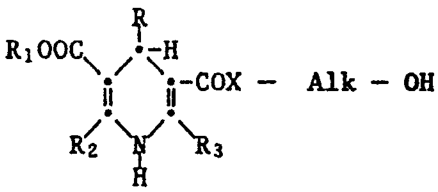

(VI)

oder einen reaktionsfähigen Ester davon, mit einer Verbindung der Formel

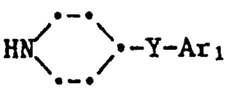

(VIa)

umsetzt, oder

e) eine Verbindung der Formel

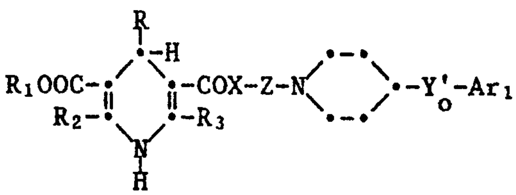

(VII)

worin Z die Bedeutung von Alk hat oder eine mittels Reduktion in die Gruppe Alk überführbare Gruppe darstellt, $Y'_o$ die Bedeutung von Y hat, oder eine mittels Reduktion in die der Bedeutung von Y entsprechende Alkylengruppe überführbare Gruppe darstellt, wobei mindestens eine der Gruppen Z und $Y'_o$ eine mittels Reduktion in die Gruppe Alk bzw. in die der Gruppe Y entsprechende Alkylengruppe überführbare Gruppe darstellt, und die andere Alk oder die Gruppe Y bedeutet, reduziert, wobei die Ausgangsstoffe der Formel II bis VII, sofern sie salzbildende Eigenschaften aufweisen, auch in Form ihrer Salze verwendet werden können, und die Gruppen R, $R_1$, $R_2$, $R_3$, X, Y, $Ar_1$ und Alk die unter der Formel I angegebenen Bedeutungen haben, und, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I in ein Salz umwandelt, und/oder, wenn erwünscht, ein erhaltenes Racematgemisch in die reinen Racemate bzw. Diastereomeren und/oder ein erhaltenes Racemat in die optischen Antipoden auftrennt.

Uéblicherweise werden die in der Verfahrensvariante a) verwendeten Ausgangsstoffe der Formel II <u>in situ</u> gebildet und der verfahrensgemässe Ringschluss kann unter den Reaktionsbedingungen für die Herstellung des Ausgangsmaterials stattfinden. So kann man die Ausgangsstoffe der Formel II und unter den Reaktionsbedingungen üblicherweise auch die entsprechenden Endprodukte der Formel I erhalten, indem man aa) eine Verbindung der Formel III, oder ein reaktionsfähiges funktionelles Derivat davon, mit einer Verbindung der Formel

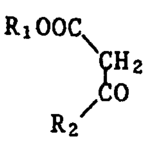

(X),

und einer Verbindung der Formel

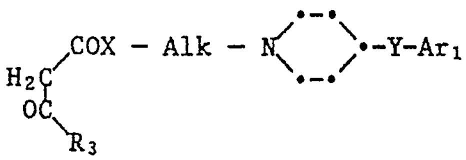

(XI),

und Ammoniak bzw. mit einer Verbindung der Formel XIII

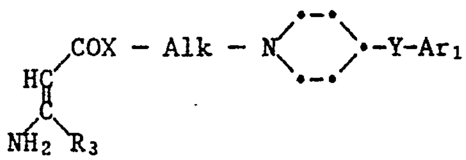

(XIII),

umsetzt, oder ab) eine Verbindung der Formel III oder ein reaktionsfähiges funktionelles Derivat davon mit einer Verbindung der Formel

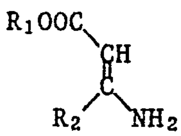

(XII),

und einer Verbindung der Formel XI oder eine Verbindung der Formel XII mit einer Verbindung der Formel XV

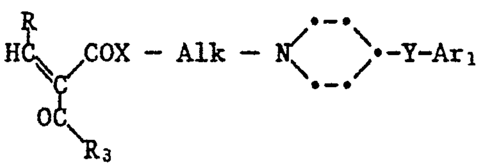

(XV),

umsetzt, oder ac) eine Verbindung der Formel III oder ein reaktionsfähiges funktionelles Derivat davon mit einer Verbindung der Formel

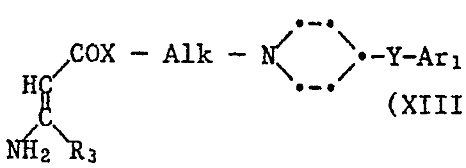

(XIII),

und einer Verbindung der Formel X oder XII oder eine Verbindung der Formel XIII mit einer Verbindung der Formel XIV

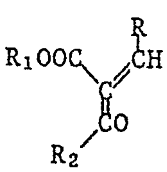

(XIV),

umsetzt, oder ad) Ammoniak mit einer Verbindung der Formel

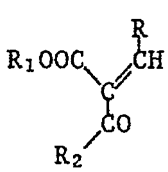

(XIV),

und einer Verbindung der Formel XI umsetzt, oder ae) Ammoniak mit einer Verbindung der Formel

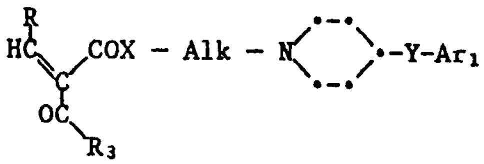

(XV),

und einer Verbindung der Formel X oder XII umsetzt, oder af) Ammoniak mit einer Verbindung der Formel

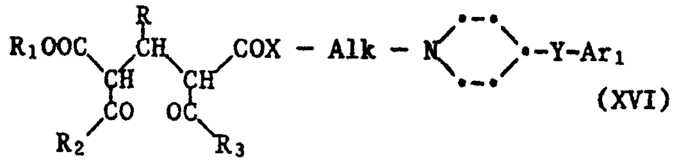

(XVI),

umsetzt, oder ag) eine Verbindung der Formel XII mit einer Verbindung der Formel XV oder der Formel

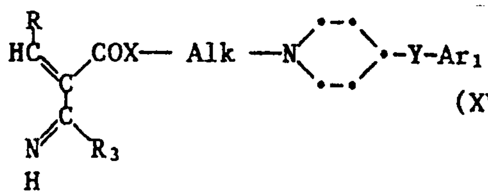

(XVII),

umsetzt, oder ah) eine Verbindung der Formel XIII mit einer Verbindung der Formel XIV oder mit einer Verbindung der Formel

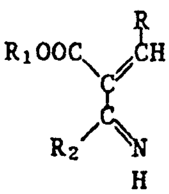

(XVIII),

umsetzt. Dabei können mit Ausnahme der Verbindung der Formel III die Verbindungen der Formeln X bis XVIII in Form von Tautomeren davon oder in Form von Tautomerengemischen verwendet werden; Ausgangsstoffe der obigen Formeln mit salzbildenden Eigenschaften können auch in Form von Salzen verwendet werden. Ferner haben in den obgenannten Verbindungen die Gruppen R, $R_1$, $R_2$, $R_3$, X, Y, $Ar_1$ und Alk die im Zusammenhang mit der Formel I gegebenen Bedeutungen.

Reaktionsfähige funktionelle Derivate des Aldehyds der Formel III sind u.a. die entsprechenden Acetale, d.h. die der Gruppe R entsprechenden Di-(veräthertes Hydroxy)-methyl-Verbindungen, wie Diniederalkyl-, z.B. Dimethyl- oder Diäthylacetale, Acylale, z.B. die entsprechenden Di-Acyloxymethyl- oder Di-Halogen-methyl-Verbindungen, wie Diniederalkanoylacylale, z.B. Diacetylacylale, oder die entsprechenden Dihalogen-, z.B. Dichlor-oder Dibrom-Verbindungen, ferner Additionsverbindungen, wie solche mit einem Alkalimetall-, z.B. Kaliumhydrogensulfit.

Das für die vorstehend beschriebenen Ringschlussreaktionen verwendete Ammoniak kann auch in Form eines, diese Verbindung in situ abgebenden Mittels, z.B. in form eines Ammoniumsalzes, wie Ammoniumacetat oder Ammoniumhydrogencarbonat, verwendet werden.

Bei der Ringschlussreaktion a), sowie den Kondensationsreaktionen aa) bis ah) zur Herstellung des üblicherweise in situ gebildeten Ausgangsmaterials für die Ringschlussreaktion, handelt es sich um Varianten der Dihyropyridinsynthese von Hantzsch. Bei der Variante aa) werden insgesamt drei Moleküle Wasser abgespalten; bei weiteren Varianten tritt teilweise an die Stelle der Wasserabspaltung eine Additionsreaktion, d.h. die Wasserabspaltung erfolgt schon bei der Herstellung von einem oder von zwei Ausgangsstoffen. Bei der Umsetzung von Verbindungen der Formel III mit Verbindungen der Formeln XIII und XII gemäss der Stufe ac), von Verbindungen der Formel XII mit Verbindungen der Formel XVII gemäss der Stufe ag), oder von Verbindungen der Formel XIII mit Verbindungen der Formel XVII gemäss Stufe ah), wird zusätzlich zu bzw. anstelle von Wasser Ammoniak abgespalten. Falls nach der Variante aa) Verbindungen der Formel I hergestellt werden sollen, in denen sich $R_2$ und $R_3$ voneinander unterscheiden, können Nebenprodukte entstehen, die in 2-und 6-Stellung die gleichen Substituenten enthalten. Durch nicht-gleichzeitiges Zusammengeben der Reaktionsteilnehmer kann die Bildung solcher Nebenprodukte indessen herabgesetzt werden, indem man einen bestimmten Reaktionsverlauf fördert, der in situgemäss einer andern Variante verläuft, da entsprechend der gestaffelten Zugabe der Reaktionskomponenten z.B. zunächst eine Verbindung der allgemeinen Formel XII oder der Formel XIII entstehen kann.

Die verfahrensgemässen Ringschluss- bzw. Kondensationsreaktionen z.B. der Varianten a) und b) werden in sich bekannter Weise durchgeführt, falls notwendig, in Gegenwart eines Kondensationsmittels, insbesondere eines basischen Kondensationsmittels, wie eines Ueberschusses einer basischen Reaktionskomponente oder eine zusätzlichen, z.B. organischen Base, wie Piperidin oder Aethyldiisopropyl-amin, oder eines Metallalkoholats, wie Alkalimetallniederalkanolats, und/oder eines geeigneten Dehydratisierungs- oder Wasser-aufnehmenden Mittels, ferner üblicherweise in Gegenwart eines inerten organischen Lösungsmittels und bei Reaktionstemperaturen im Bereich von etwa Raumtemperatur bis etwa 150°C, insbesondere bei Siedetemperatur des Lösungsmittels. Gegebenenfalls erfolgt die Umsetzung in einer Inertgas-, z.B. Stickstoffatmosphäre, und/oder, z.B. bei Verwendung eines niedrigsiedenden Lösungsmittels und/oder von Ammoniak, im geschlossenen Gefäss unter erhöhtem Druck.

Die in den Verfahrensvarianten verwendeten Ausgangsstoffe sind bekannt oder können nach an sich bekannten Verfahren hergestellt werden.

So lassen sich z.B. Ausgangsstoffe der Formel XI durch Umsetzung von Verbindungen der Formel

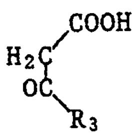 (XIa)

mit Verbindungen der Formel

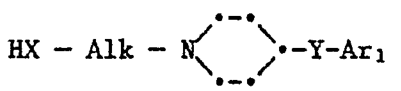 (XIb)

auf übliche Weise herstellen. Anstelle von Carbonsäuren der Formel XIa können auch funktionelle Derivate davon, wie entsprechende Carbonsäureanhydride, insbesondere gemischte Anhydride, wie solche mit Niederalkancarbonsäuren, etwa Ameisensäure, ferner Säurehalogenide, z.B. entsprechende Chloride, Bromide, ferner Säureazide, weiter aktivierte Ester, z.B. Cyanomethylester, verwendet werden. Diese können, gegebenenfalls in Gegenwart von Kondensationsmitteln, durch Umsetzung mit einer Verbindung der Formel XIb, freie Carbonsäuren der Formel XIa auch durch Umsetzen mit Verbindungen der Formel XIb, worin anstelle der Gruppe HX- die Azidogruppe steht, zu Verbindungen der Formel XI umgesetzt werden. Carbonsäuren der Formel XIa können auch als Salze, insbesondere als Alkalimetall-oder Erdalkalimetallsalze mit reaktionsfähigen Estern von Alkoholen der Formel XIb, worin X für Sauerstoff steht, wie entsprechenden Halogeniden, z.B. Chloriden, Bromiden oder Jodiden, oder organischen Sulfonsäureestern, z.B. Niederalkansulfonsäure-oder Arensulfonsäureestern, wie Methansulfonsäure- bzw. p-Toluol sulfonsäureestern, zu entsprechenden Carbonsäureestern umgesetzt werden, oder man hydrolysiert entsprechende hydrolysierbare Iminoester, wie entsprechende Iminoniederalkylester, zu den Estern. Iminoester dieser Art sind beispielsweise aus den Verbindungen der Formel XIa entsprechenden Nitrilen der Formel

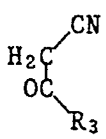 (XIc)

durch Umsetzung mit Verbindungen der Formel XIb, worin X für Sauerstoff steht, in Gegenwart eines sauren Kondensationsmittels, etwa Chlorwasserstoff, in einem geeigneten Lösungsmittel, etwa eines solchen inerten Charakters, wie eines Aromaten, z.B. Benzol, auf übliche Weise zugänglich.

Verbindungen der Formel XIb wiederum sind auf an sich bekannte Weise durch Umsetzung von Verbindungen der Formel

HX-Alk-A (XId),

worin A eine geeignete Abgangsgruppe, z.B. eine reaktionsfähige veresterte Hydroxygruppe, wie etwa Halogen, z.B. Chlor, Brom oder Jod, oder eine Sulfonylgruppe, z.B. eine Arylsulfonyloxy- wie p-Toluolsulfonyloxygruppe, darstellt mit Verbindungen der Formel

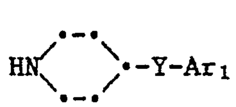 (XIe),

zweckmässigerweise in Gegenwart eines basischen Kondensationsmittels, wie eines Oxids, Hydroxids oder Carbonats eines Alkalimetall- oder Erdalkalimetalls, wie Natriumhydroxid oder Calciumcarbonat, und

üblicherweise in Gegenwart eines Lösungsmittels, etwa eines Niederalkanols, wie Aethanol, bei erhöhter oder erniedrigter Temperatur zugänglich. Verbindungen der Formel XIe können auch in Form ihrer Metallderivate eingesetzt werden, worin das am Stickstoff stehende Wasserstoffatom durch ein geeignetes Metall, etwa Lithium oder Kalium ersetzt ist. In solchen Fällen erfolgt die beschriebene Umsetzung mit Verbindungen der Formel XId in einem inerten wasserfreien Lösungsmittel, etwa eines solchen ätherartigen Charakters, wie Tetrahydrofuran, oder eines aromatischen Lösungsmittels, etwa Toluol.

Der Formel XIe entsprechende Metallverbindungen sind in üblicher Weise, z.B. durch Umsetzung mit einer geeigneten Alkalimetallorganischen Verbindung, etwa Butyllithium in einem wasserfreien Lösungsmittel, wie Tetrahydrofuran, zweckmässigerweise unter einem Schutzgas, z.B. Argon, zugänglich, wobei die im Reaktionsgemisch enthaltene Metallverbindung, ohne diese zu isolieren, für die oben beschriebene Umsetzung verwendet werden kann.

Verbindung der Formel XIe wiederum sind auf an sich bekannte Weise herstellbar, etwa aus solchen Ausgangsstoffen, die anstelle des Piperidinringes eine geeignete in einen Piperidinring überführbare Gruppe enthält. Solche Gruppe sind etwa solche, die z.B. mittels Reduktion oder mittels ringschliessender Kondensation den Piperidinring bilden, etwa ein entsprechendes 2-Piperidon, welches mittels eines geeigneten Reduktionsmittels, wie Lithiumalumniumhydrid, in einen Ausgangsstoff der Formel XIe umgewandelt werden kann. Oder man geht z.B. von einem entsprechenden 1,5-Di-halogenpentan, etwa einem entsprechenden 1,5-Dibrompentan aus, welches mittels Ammoniak, gegebenenfalls im geschlossenen Gefäss und unter Druck einen Ausgangsstoff der Formel XIe ergibt.

Die Umsetzung von freien Carbonsäuren der Formel XIa mit Verbindungen der Formel XIb erfolgt vorteilhaft in Gegenwart eines sauren, die Wasserabspaltung fördernden Katalysators, wie einer Protonensäure, z.B. Chlorwasserstoff-, Bromwasserstoff-, Schwefel-, Phosphor- oder Borsäure, Benzolsulfon- oder Toluolsulfonsäure, oder einer Lewissäure, z.B. von Bortrifluorid-Aetherat, in einem Ueberschuss des eingesetzten Alkohols und/oder in einem inertem Lösungsmittel, erforderlichenfalls unter destillativer, z.B. azeotroper, Entfernung des bei der Reaktion freigesetzten Wassers. Weiter kann man die Umsetzungen auch in Gegenwart von wasserbindenden Kondensationsmitteln, wie geeignet substituierten Carbodiimiden, z.B. N,N'-Diäthyl-, N,N'-Dicyclohexyl-oder N-Aethyl-N'-(3-dimethylaminopropyl)-carbodiimid, in inerten organischen Lösungsmitteln, durchführen. Gemischte Anhydride, insbesondere Säurehalogenide, werden beispielsweise in Gegenwart säurebindender Mittel, z.B. organischer, insbesondere tertiärer Stickstoffbasen, wie Triäthylamin, Aethyldiisopropylamin oder Pyridin, oder auch anorganischen Basen, z.B. Alkalimetall- oder Erdalkalimetallhydroxiden oder -carbonaten, wie Natrium- Kalium-oder Calciumhydroxid bzw. -carbonat, mit Alkoholen oder mit Alkoholaten, z.B. Alkalimetall-niederalkanolaten, umgesetzt.

Die Umsetzungen von reaktionsfähigen Estern, z.B. Cyanmethyl- oder Pentachlorphenylestern, mit Verbindungen der Formel XIb werden beispielsweise in einem gegenüber den Reaktionsteilnehmern inerten Lösungsmittel im Temperaturbereich von etwa 0°C bis etwa 120°C, vorzugsweise bei Raumtemperatur bis etwa 60°C, durchgeführt.

Die Hydrolyse von Iminoesterausgangsstoffen, erfolgt beispielsweise mittels wasserhaltiger Mineralsäuren, wie Salzsäure oder Schwefelsäure, wobei man z.B. die bei der Addition von Chlorwasserstoff an Nitrile und Umsetzung mit wasserfreien Alkoholen, insbesondere unsubstituierten oder substituierten Niederalkanolen, erhaltenen Iminoester-salze, z.B. -hydrochloride, nach Zusatz von Wasser direkt zu den entsprechenden Estern hydrolysieren kann. Man kann z.B. auch aus einem Gemisch von Nitril, Alkohol und Schwefelsäure mit geeignetem Wassergehalt ohne Isolierung des in situ entstandenen Iminoesters die gewünschte Esterverbindung der Formel XI enthalten.

Ausgangsstoffe der Formeln XIII, XV, XVI und XVII können ausgehend von entsprechenden Ausgangsmaterialien auf analoge und übliche Weise hergestellt werden.

Ausgangsstoffe der Formel IV werden analog wie bei der Herstellung von Ausgangsstoffen der Formel II beschrieben in situ gebildet, und der verfahrensgemässe Ringschluss zu den Endprodukten der Formel I kann unter den für die Herstellung des Ausgangsmaterials gegebenen Bedingungen im gleichen Reaktionsansatz erfolgen. Dementsprechend sind Ausgangsstoffe der Formel IV durch Umsetzung von Verbindungen der Formel XI mit solchen der Formel X und Ammoniak, oder von Verbindungen der Formel XI mit solchen der Formel XII, oder von Verbindungen der Formel XIII mit solchen der Formel X oder XII zugänglich, wobei solche Umsetzung üblicherweise in einem geeigneten Lösungsmittel, etwa einem Niederalkanol, wie Aethanol, gegebenenfalls bei erhöhter oder erniedrigter Temperatur, zweckmässigerweise unter einem Schutzgas, wie Stickstoff, durchgeführt werden.

Ausgangsstoffe der Formel V können entsprechend dem bzw. den in ihnen enthaltenen Rest(en) $Ac^{o}$ und/oder $Ac_{1}^{o}$, beispielsweise Carbonsäuren ($Ac^{o}$ und/oder $Ac_{1}^{o}$ ist Carboxyl), Carbonsäureanhydride, insbesondere gemischte Anhydride, wie Säurehalogenide, z.B. -chloride oder -bromide oder Azide ($Ac^{o}$und/oder $Ac_{1}^{o}$ ist Halogencarbonyl, z.B. Chlor-oder Bromcarbonyl oder Azidocarbonyl), weiter aktivierte Ester, z.B. Cyanomethylester ($Ac^{o}$ und/oder $Ac_{1}^{o}$ ist Cyanmethoxycarbonyl) sein; diese können, gegebenenfalls in Gegenwart von Kondensationsmitteln, durch Behandeln mit einem entsprechenden Alkohol, in die entsprechenden Ester umgewandelt werden, beispielsweise zur Umwandlung der Gruppe $Ac^{o}$ in eine der Gruppe $-COOR_1$ entsprechende Carboxylestergruppe durch Umsetzung mit einem der Bedeutung von $R_1$ entsprechenden unsubstituierten oder substituierten Niederalkanol, oder einem reaktionsfähigen Derivat davon, z.B. einem entsprechenden Alkoholat, freie Carbonsäuren auch durch Umsetzen mit geeigneten Diazo-Verbindungen, wie unsubstituierten oder substituierten Diazoniederalkanen, wobei

Verbindungen der Formel I entstehend, welche die Gruppe -$COOR_1$ enthalten.

Zur Umwandlung der Gruppe Ac$_1^o$ in die Gruppe der oben definierten Formel Va setzt man einen Ausgangsstoff der Formel V mit einer Verbindung der Formel XIb in üblicher Weise um. Carbonsäureester der angegebenen Art, worin X für Sauerstoff steht, können ebenfalls erhalten werden, wenn als Ausgangsstoffe Salze, insbesondere Alkalimetall- oder Erdalkalimetallsalze der freien Carbonsäuren verwendet und diese mit reaktionsfähigen Estern von den Verbindungen der Formel XIb entsprechenden Alkoholen, worin X Sauerstoff ist, wie entsprechenden Halogeniden, z.B. Chloriden, Bromiden oder Iodiden, oder organischen Sulfonsäureestern, z.B. Niederalkansulfonsäure-oder Arensulfonsäureestern, wie Methansulfonsäure- bzw. p-Toluolsulfonsäureestern, behandelt werden, oder wenn man entsprechende hydrolysierbare Iminoester, wie entsprechende Iminoniederalkylester, zu den Estern hydrolysiert.

Iminoester dieser Art sind z.B. aus Ausgangsstoffen der Formel V, worin Ac$^o$ und/oder Ac$_1^o$ die Cyanogruppe darstellt, durch Umsetzung mit einem der Bedeutung von $R_1$ entsprechenden Niederalkanol und/oder einem Alkohol der Formel XIb, worin X Sauerstoff ist, in Gegenwart eines sauren Kondenstionsmittels, wie Chlorwasserstoff oder konz. Schwefelsäure zugänglich.

Die Umsetzung von frein Carbonsäuren mit Alkoholen, wie unsubstituierten oder substituierten Niederalkanolen oder mit Verbindungen der Formel XIb, erfolgt vorteilhaft in Gegenwart eines sauren, die Wasserabspaltung fördernden Katalysators, wie einer Protonensäure, z.B. Chlorwasserstoff-, Bromwasserstoff-, Schwefel-, Phosphor- oder Borsäure, Benzolsulfon- oder Toluolsulfonsäure, oder einer Lewissäure, z.B. von Bortrifluorid-Aetherat, in einem Ueberschuss des eingesetzten Alkohols bzw. der Verbindung der Formel XIb und/oder in einem inerten Lösungsmittel, erforderlichenfalls unter destillativer, z.B. azeotroper, Entfernung des bei der Reaktion freigesetzten Wassers. Weiter kann man die Umsetzungen auch in Gegenwart von wasserbindenden Kondensationsmitteln, wie geeignet substituierten Carbodiimiden, z.B. N,N'-Diäthyl, N,N'-Dicyclohexyl-oder N-Aethyl-N'-(3-dimethylaminopropyl)-carbodiimid, in inerten organischen Lösungsmitteln, durchführen. Gemischte Anhydride, insbesondere Säurehalogenide, werden beispielsweise in Gegenwart säurebindender Mittel, z.B. organischer, insbesondere tertiärer Stickstoffbasen, wie Triäthylamin, Aethyldiisopropylamin oder Pyridin, oder auch anorganischen Basen, z.B. Alkalimetall- oder Erdalkalimetallhydroxiden oder -carbonaten, wie Natrium-, Kalium- oder Calciumhydroxid bzw. -carbonat, mit Alkoholen oder mit Alkoholaten, z.B. Alkalimetall-niederalkanolaten, umgesetzt.

Die Umsetzungen von der Formel V entsprechenden reaktionsfähigen Estern, z.B. Cyanmethyl-, Benztriazol-1-yl oder Pentachlorphenylestern, mit der Bedeutung von $R_1$ entsprechenden Niederalkanolen bzw. mit Verbindungen der Formel XIb werden beispielsweise in einem gegenüber den Reaktionsteilnehmern inerten Lösungsmittel im Temperaturbereich von etwa 0°C bis etwa 120°C, vorzugsweise bei Raumtemperatur bis etwa 60°C, durchgeführt.

Die Hydrolyse von Iminoesterausgangsstoffen, erfolgt beispielsweise mittels wasserhaltiger Mineralsäuren, wie Salzsäure oder Schwefelsäure, wobei man z.B. die bei der Addition von Chlorwasserstoff an Nitrile und Umsetzung mit wasserfreien Alkoholen, insbesondere unsubstituierten oder substituierten Niederalkanolen, erhaltenen Iminoester-salze, z.B. -hydrochloride, nach Zusatz von Wasser direkt zu den entsprechenden Estern hydrolysieren kann. Mann kann z.B. auch aus einem Gemisch von Nitril, Alkohol und Schwefelsäure mit geeignetem Wassergehalt ohne Isolierung des in situ entstandenen Imidoesters die gewünschte Esterverbindung der Formel I erhalten.

Ausgangsstoffe der Formel V mit freier Carboxylgruppe Ac$^o$ und/oder Ac$_1^o$ können z.B. erhalten werden, indem man den entsprechenden 2-Cyanoäthylester herstellt, wobei man z.B. in einem der vorstehende beschriebenen Verfahren ab) oder ag) eine Verbindung der Formel XII einsetzt, in der anstelle der Gruppe -$COOR_1$ eine 2-Cyanäthoxycarbonylgruppe steht; z.B. kann man einen die Gruppe $R_2$ enthaltenden 3-Aminocrotonsäure-2-cyanoäthylester mit den übrigen Reaktionskomponenten umsetzen, und anschliessend die so erhaltene 2-Cyano äthylester-Verbindung unter milden Bedingungen, z.B. mittels wässrigem oder wässerig-niederalkanolischem 1-n. Natriumhydroxid bei Raumtemperatur, zur freien Carbonsäure spalten. Letztere kann, falls notwendig, in an sich bekannter Weise in die gewünschten reaktionsfähigen funktionellen Derivate übergeführt werden.

Die als Ausgangsstoffe für die Verfahrensvariante c) ebenfalls in Betracht kommenden Nitrilverbindungen der Formel V können z.B. analog zu einer der Verfahrensvarianten aa) bis ah) hergestellt werden, indem man Ausgangsstoffe verwendet, die anstelle des Restes -$COOR_1$ bzw. der Gruppe der Formel XIb eine Cyangrupe enthalten, wie beispielsweise anstelle einer Verbindung der Formel XII die Gruppe $R_2$ enthaltendes 3-Aminocrotonitril.

Die für die Verfahrensvariante d) benötigen Ausgangsstoffe der Formel VI können auf an sich bekannte Weise hergestellt werden, z.B. analog den in den Verfahrensstufen aa) bis ah) beschriebenen Umsetzungen, wobei anstelle der Verbindungen XI, XIII, XV, XVI oder XVII solche Ausgangsmaterialien eingesetzt werden, die anstelle der Gruppe der Formel

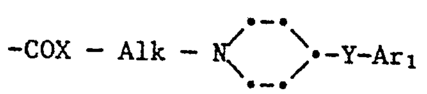 (VIa),

die Gruppe der Formel -COX-Alk-OH (VIb) bzw. einen reaktionsfähigen Ester, davon aufweisen.

Reaktionsfähige Ester sind z.B. solche mit einer Halogenwasserstoffsäure, z.B. Salzsäure, oder einer organischen Sulfonsäure, wie einer Arylsulfonsäure, z.B. p-Toluolsulfonsäure gebildete Ester, in denen die Hydroxygruppe also z.B. durch Halogen, wie Chlor oder Brom, oder z.B. durch Arylsulfonyloxy, wie p-Toluolsulfonyloxy ersetzt ist. So kann z.B. ein Ausgangsstoff der Formel

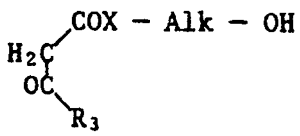

(VIc)

analog den für die Herstellung von Ausgangsstoffen der Formel XI beschriebenen Methoden, z.B. durch Umsetzung von Verbindungen der Formel XIa mit Verbindungen der Formel HX-Alk-OH (VId) oder einem reaktionsfähigen Ester, etwa einem Halogenid, wie Bromid oder Jodid, wie beschrieben, erhalten werden. Oder man setzt ein Nitril der Formel XIc mit einer Verbindung der Formel VId, worin X Sauerstoff ist, in Gegenwart eines sauren Kondensationsmittels, etwa Chlorwasserstoff, zum entsprechenden Iminoester-Salz um, der dann mittels Wasser im sauren Medium, z.B. wie oben beschrieben, zum entsprechenden Carbonsäureester hydrolysiert wird.

Die obigen Reaktionen und ebenso die Verfahrensvariante d) können unter an sich bekannten Reaktionsbedingungen durchgeführt werden, in Ab- oder üblicherweise Anwesenheit von Lösungs- oder Verdünnungsmitteln, je nach Art der Reaktion und/oder Reaktionsteilnehmer bei erniedrigter oder erhöhter Temperatur, z.B. im Temperaturbereich von etwa -10° C bis etwa 150° C, unter atmosphärischem Druck oder in einem geschlossenen Gefäss, gegebenenfalls unter Druck, und/oder in einer inerten Atmosphäre, z.B. unter einer Stickstoffatomosphäre. Falls erforderlich, kann die Umsetzung unterstützend in Gegenwart einer Base durchgeführt werden.

Die für die Verfahrensvariante e) benötigten mittels Reduktion in die Gruppe Alk überführbaren Gruppen Z bzw. die mittels Reduktion in die der Gruppe Y entsprechende Alkylengruppe Gruppen enthalten Doppel- und/oder Dreifachbindungen und/oder Carbonyl- bzw. Thiocarbonylgruppen im entsprechenden Alkylenrest.

Die Reduktion von ungesättigten Gruppen zur Kohlenstoff-Kohlenstoff-Einfachbindung erfolgt beispielsweise mittels aktiviertem Wasserstoff, wie Wasserstoff in Gegenwart eines Hydrierkatalysators, z.B. eines Nickel-, Platin- oder Palladiumkatalysators, während die Reduktion von Carbonylgruppen zur Methylengruppe mittels Wasserstoff in Gegenwart z.B. eines Kupferchromit-Katalysators oder nach der Methode von Clemmensen, z.B. mittels gegebenenfalls analgamiertem Zink in einer Mineralsäure, etwa Salzsäure, erfolgen kann. Die Reduktion von Thiocarbonylgruppen kann auf ähnliche Weise erfolgen, z.B. unter Verwendung eines schwefelfesten Katalysators. Die Reduktion der vorstehend genannten Gruppen kann ferner mittels eines Hydridreduktionsmittels, z.B. Natriumborhydrid oder Diboran, erfolgen. Bei dessen Reduktionen muss, sofern erwünscht, Sorge dafür getragen werden, dass andere gegen Reduktion empfindliche Gruppen, z.B. ungesättigte Gruppen oder Nitrogruppen nicht angegriffen werden, z.B. durch Auswahl des geeigneten Reduktionsmittels, dessen für die durchzuführende Reduktion erforderliche Dosierung und/oder geeignete Verfahrensbedingungen, z.B. erhöhte oder erniedrigte Temperatur und/oder durch Anwendung eines geeigneten Lösungsmittels. Diese Umsetzungen werden in an sich bekannter Weise durchgeführt, üblicherweise in Anwesenheit von Lösungs- und Verdünnungsmitteln, je nach Art der Reaktion und/oder Reaktionsteilnehmer bei erniedrigter oder erhöhter Temperatur, z.B. im Temperaturbereich von etwa -15°C bis etwa 120°C unter normalem Druck oder im geschlossenen Gefäss, gegebenenfalls unter Druck und/oder unter einem Schutzgas, etwa Stickstoff.

Ausgangsstoffe der Formel VII können, sofern sie neu sind, nach an sich bekannten Methoden hergestellt werden, z.B. analog den unter a) bis d) beschriebenen Verfahren. So kann man z.B. analog den in den Stufen aa) bis ah) beschriebenen Reaktionsschritten verfahren, wenn man anstelle der Verbindungen XI, XIII, XV, XVI oder XVII solche Verbindungen einsetzt, die anstelle der oben definierten Gruppe Va die Gruppe der Formel

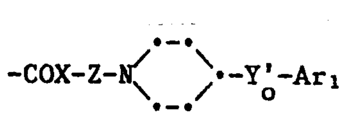

(VIIa)

enthält.

So kann z.B. ein Ausgangsstoff der Formel

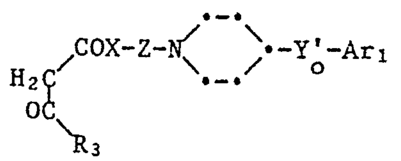

(VIIb),

analog den für die Herstellung von Verbindungen der Formel XI beschriebenen Methoden, etwa durch Umsetzung von Verbindungen der Formel XIa oder reaktionsfähigen Derivaten davon, z.B. Anhydriden, Säurehalogeniden, etwa Chloriden, Aziden, gemischten Estern wie Cyanomethylestern oder Pentachlorphenolestern, hergestellt werden. Carbonsäuren der Formel XIa können auch als Salze, insbesondere als Alkalimetall- oder Erdalkalimetallsalze mit reaktionsfähigen Estern der Formel

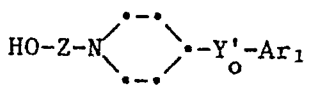

(VIIc),

wie entsprechenden Halogeniden, z.B. Chloriden, Bromiden oder Jodiden, oder organischen Sulfonsäureestern, etwa die mit Niederalkansulfonsäuren, wie Methansulfonsäure, oder Arylsulfonsäuren, wie Benzolsulfonsäure, zu entsprechenden Carbonsäureestern umgesetzt werden. Alkohole der Formel VIIc können andererseits mti Nitrilen der Formel XIc in üblicher Weise in Gegenwart saurer Kondensationsmittel, wie einer Mineralsäure etwa Chlorwasserstoff- oder Schwefelsäure, zu den Salzen entsprechender Iminoester umgesetzt werden, die dann mittels Wasser, wie beschrieben, zu den Carbonsäureestern hydrolysiert werden. Diese Umsetzungen werden unter üblichen und an sich bekannten Reaktionsbedingungen vorgenommen. Ausgangsstoffe der Formel VIIc wiederum, sind, soweit sie neu sind, auf übliche Weise, z.B. durch Umsetzung von Verbindungen der Formel HO-Z-A (VIId), worin A eine geeignete Abgangsgruppe, z.B. Halogen, wie Chlor, Brom oder Jod oder eine Sulfonyloxy- wie p-Toluolsulfonyloxygruppe ist, mit Verbindungen der Formel XIe auf übliche Weise zugänglich.

Die obigen Reaktionen können unter an sich bekannten Reaktionsbedingungen durchgeführt werden, in Ab- oder üblicherweise Anwesenheit von Lösungs- oder Verdünnungsmitteln, je nach Art der Reaktion und/oder Reaktionsteilnehmer bei erniedrigter oder erhöhter Temperatur, z.B. im Temperaturbereich von etwa -10°C bis etwa 150°C, unter atmosphärischem Druck oder in einem geschlossenen Gefäss, gegebenenfalls unter Druck, und/oder in einer inerten Atmosphäre, z.B. unter einer Stickstoffatmosphäre.

Verfahrensgemäss erhältliche Verbindungen der Formel I können in an sich bekannter Weise in andere Verbindungen der Formel I umgewandelt werden, etwa durch Umwandlung von in Verbindungen der Formel I enthaltenen Substituenten in andere von der Formel I umfasste Substituenten.

So kann man z.B. eine veresterte Carboxygruppe $-COOR_1$ durch Umesterung in eine andere Estergruppe überführen. Dabei verwendet man vorzugsweise entsprechende Alkoholverbindungen, die einen Siedepunkt aufweisen, der deutlich über demjenigen des Alkohols der veresterten Gruppe in der umzuwandelnden Verbindung der Formel I liegt, und führt die Reaktion z.B. in einem Ueberschuss der Hydroxyverbindung und/oder einem inerten organischen, vorzugsweise ebenfalls deutlich über dem Alkohol der veresterten Gruppe siedenden Lösungsmittel, vorzugsweise in Gegenwart eines Katalysators, z.B. eines Alkalimetall-niederalkanolats, wie Natrium- oder Kalium-methanolat oder -äthanolat, in der Wärme und üblicherweise unter Abdestillieren des freigesetzten Akohols durch.

Verbindungen der Formel I, worin Y für die Gruppe -(C=O)- steht, kann man in üblicher Weise mittels Reduktion,in Verbindungen der Formel I umwandeln, worin Y, gegebenenfalls über die Gruppe -CHOH-als Zwischenstufe, die Gruppe $-CH_2-$ darstellt, wobei gegebenenfalls vorhandene ungesättigte Gruppen in die gesättigten, bzw. Dreifachbindungen enthaltene Gruppe in solche mit Zweifachbindungen umgewandelt werden.

Für die Reduktion kann man z.B. katalytisch aktivierten Wasserstoff, etwa Wasserstoff in Gegenwart eines Hydrierkatalysators, wie eines Edelmetallkatalysators, z.B. Platin oder Palladium, ferner Raney-Nickel verwenden. Ebenso kann ein geeignetes Hydridredutionsmittel, etwa Diboran, oder Natriumborhydrid, ferner Lithiumaluminiumhydrid in einem geeigneten Lösungsmittel, etwa eines solchen ätherartigen Charakters, wie Tetrahydrofuran, eingesetzt werden.

Ferner kann man Verbindungen der Formel I, in welchen R und/oder $Ar_1$ geeignete azaheterocyclische Gruppen darstellen, deren Ringstickstoffatome N-oxidiert werden können, in entsprechende N-Oxide umwandeln. Die Oxidation kann in an sich bekannter Weise durchgeführt werden, z.B. durch Behandeln mit organischen Persäuren, wie Niederalkanpersäuren oder Arenpersäuren, wie gegebenenfalls geeignet substituierten Perbenzoesäuren, z.B. Peressig- oder 3-Chlorperbenzoesäure, vorzugsweise bei Zimmertemperatur oder etwas darüber liegender Reaktionstemperatur, oder mit wässrigem Wasserstoffperoxid, z.B. bei Temperaturen von bis zu 100 C, in Gegenwart oder Abwesenheit von Niederalkansäuren, z.B. Essigsäure. Dabei muss insbesondere bei Verwendung von Persäuren, darauf geachtet werden, dass keine Ueberoxidation aufgrund einer zu langen Reaktionsdauer eintreten kann.

Je nach Reaktionsbedingungen könen Verbindungen der Formel I freier Form oder in Form von Salzen erhalten werden.

So können erhaltene Säureadditionssalze in an sich bekannter Weise. z.B. durch Behandeln mit einer Base. wie einem Alkalimetallhydroxid, in die freien Verbindungen oder z.B. durch Behandeln mit geeigneten Säuren oder Derivaten davon in andere Salze umgewandelt werden. Erhaltene freie Verbindungen der Formel I können, z.B. durch Behandeln mit Säuren oder entsprechenden Anionenaustauschern, in ihre Salze umgewandelt werden.

Infolge der engen Beziehung zwischen den Verbindungen der Formel I in freier Form und in Form von Salzen, sind im Vorausgegangenen und nachfolgend unter den freien Verbindungen oder ihren Salzen sinn-und zweckgemäss gegebenenfalls auch die entsprechenden Salze bzw. freien Verbindungen zu verstehen.

Die Verbindungen der Formel I, einschliessliche deren Salze können auch in Form ihrer Hydrate erhalten werden, oder ihre Kristalle können z.B. das zur Kristallisation verwendete Lösungsmittel einschliessen.

Die Verbindungen der Formel I können, je nach Verfahrensreaktion und/oder Art der Ausgangsstoffe, in form von Racematgemischen, Racematen oder optischen Antipoden erhalten werden.

Erhaltene Racematgemische können auf Grund der physikalisch-chemischen Unterschiede der Racemate in bekannter Weise in die reinen Racemate bzw. Diastereomeren aufgetrennt werden, beispielsweise durch Chromatographie und/oder fraktionierte Kristallisation.

Racemate lassen sich nach an sich bekannten Methoden in die optischen Antipoden zerlegen, beispielsweise durch Umkristallisation aus einem optisch aktiven Lösungsmittel, mit Hilfe von geeigneten Mikroorganismen oder durch Umsetzen einer Verbindung der Formel I mit salz-bildenden, z.B. basischen, Eigenschaften mit einem optisch aktiven, salzbildenden Mittel, wie einer optisch aktiven Säure, und Trennen der auf diese Weise erhaltenen Gemische von Salzen, z.B. auf Grund ihrer verschiedenen Löslichkeiten, in die diastereomeren Salze, aus denen die Antipoden, z.B. durch Behandeln mit einer Base, freigesetzt werden können.

Optische Antipoden von neutralen Verbindungen der Formel I kann man z.B. auch gemäss Verfahren c) unter Verwendung einer optisch aktiven Säure der Formel V ($Ac^o$ bedeutet Carboxy, $Ac_1{}^o$ ist verestertes oder amidiertes Carboxy) erhalten, wobei man diese z.B. aus der entsprechenden racemischen Säure in üblicher Weise, z.B. durch Salzbildung mit einer optisch aktiven Base, Trennung der diastereomeren Salze und Freisetzung der optisch aktiven Säure und deren Umwandlung in eine der Formel I entsprechende, die Gruppe $-COOR_1-$ enthaltende Verbindung erhält.

Ferner kann man z.B. Verbindungen der Formel I, mit der Gruppe $-COOR_1-$ unter Verwendung eines optisch aktiven Alkohols nach dem oben beschriebenen Verfahren umestern und das so erhältliche Diastereomerengemisch, z.B. mittels fraktionierter Kristallisation, in die Antipoden auftrennen.

Vorteilhafterweise isoliert man aus einem Diastereomerengemisch bzw. Racemat das pharmakologisch aktivere Diastereomere bzw. den aktiveren Antipoden.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivats, z.B. Salzes, und/oder seiner Racemate bzw. Antipoden verwendet oder unter den Reaktionsbedingungen bildet.

Salze von Ausgangsstoffen mit salzbildenden basischen Eigenschaften sind z.B. solche mit Mineralsäuren, etwa Salzsäure, Schwefelsäure oder Phosphorsäure, oder mit organischen Säuren, z.B. Essigsäure.

Bei den Verfahren der vorliegenden Erfindung werden vorzugsweise solche Ausgangsstoffe verwendet, welche zu den eingangs als besonders wertvoll geschilderten Verbindungen führen. Neue Ausgangsstoffe und Zwischenprodukte sowie Verfahren zur deren Herstellung bilden ebenfalls einen Gegenstand der vorliegenden Erfindung.

Die Erfindung betrifft ebenfalls die Verwendung der Verbindungen der Formel I oder von pharmazeutisch verwendbaren Salzen von solchen Verbindungen mit salzbildenden Eigenschaften, insbesondere als pharmakologisch, in erster Linie als Coronardilatatoren und Antihy pertensiva zur Behandlung von cardiovasculären Krankheitszuständen, wie Angina pectoris und ihren Folgen, zentralen und peripheren Durchblutungsstörungen, hohem Blutdruck, Arrythmien und Herzinsuffizienz wirksame Verbindungen. Dabei kann man sie, vorzugsweise in Form von pharmazeutischen Präparaten, in einem Verfahren zur prophylaktischen und/oder therapeutischen Behandlung des tierischen und menschlichen Körpers, insbesondere zur Behandlung von cardiovasculären Krankheitszuständen, wie Angina pectoris und ihren Folgen, Gefässspasmen, hohem Blutdruck und Herzinsuffizienz verwenden.

Die Dosierung des Wirkstoffs, der allein oder zusammen mit dem üblichen Träger- und Hilfsmaterial verabreicht wird, hängt von der zu behandelnden Spezies, deren Alter und individuellem Zustand, sowie der Verabreichungsweise ab. Die täglichen Dosen liegen z.B. für Mammalien mit einem Körpergewicht von etwa 70 kg, je nach Art der Erkrankung, individuellem Zustand und Alter, vorzugsweise zwischen etwa 10 und 100 mg, inbesondere zwischen etwa 20 mg und etwa 80 mg, und speziell etwa bei 25 mg bis etwa 75 mg bei oraler Verabreichung.

Die Erfindung betrifft im weiteren pharmazeutische Präparate, die Verbindungen der Formel I oder pharmazeutisch verwendbare Salze von solchen Verbindungen mit salzbildenden Eigenschaften als Wirkstoffe enthalten, sowie Verfahren zu ihrer Herstellung.

Bei den erfindungsgemässen pharmazeutischen Präparaten handelt es sich um solche zur enteralen, wie

peroralen oder rektalen, weiter zur sublingualen. sowie zur parenteralen Verabreichung an Warmblüter. Entsprechende Dosiseinheitsformen, insbesondere zur peroralen und/oder sublingualen Verabreichung, z.B. Dragées, Tabletten oder Kapseln, enthalten vorzugsweise von etwa 10 mg bis etwas 100 mg, insbesondere von etwa 20 mg bis etwa 80 mg einer Verbindung der Formel I oder eines pharmazeutisch annehmbaren Salzes einer zur Salzbildung befähigten entsprechenden Verbindungen zusammen mit pharmazeutisch verwendbaren Trägerstoffen.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose und/oder, wenn erwünscht, Sprengmittel, wie die obgenannten Stärken, ferner Carboxymethylstärke, quervernetztes Polyvinyl-pyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, mit Magnesium- oder Calciumstearat, und/oder Polyäthylenglykol. Dragée-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyäthylenglykol und/oder Titandioxid enthalten, oder Lacklösungen in geeigneten organischen Lösungsmitteln oder Lösungmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eine Granulats, z.B. im Gemisch mit Füllstofffen, wie Lactose, Bindemitteln, wie Stärken und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können. Bevorzugt sind u.a. Kapseln, die sowohl leicht zerbissen werden können, um durch sublinguale Aufnahme des Wirkstoffes eine möglichst rasche Wirkung zu erzielen, als auch unzerkaut geschluckt werden können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffes mit einer Grundmasse enthalten; als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffes in wasserlöslicher Form, z.B. eines wasserlöslichen Salzes, ferner Suspensionen des Wirkstoffes, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat, oder Trigylceride verwendet, oder wässrige Injektionssuspensionen, welche viskositätserhöhende Stoffe, z.B. Natriumcarboxymethylcellulose, Sorbit und/oder Dextran und gegebenenfalls Stabilisatoren enthalten.

Die pharmazeutischen Präparate der vorliegenden Erfindung können in an sich bekannter Weise, z.B. mittels konventioneller Misch-, Granulier-, Dragier-, Lösungs-, oder Lyophilisierungsverfahren hergestellt werden. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfstoffen, zu Tabletten oder Dragée-Kernen verarbeitet.

Die nachfolgenden Beispiele illustrieren die oben beschriebene Erfindung; sie sollen jedoch diese in ihrem Umfang in keiner Weise einschränken. Temperaturen sind in Celsiugraden angegeben.

Beispiel 1: Zu einem Gemisch von 12 ml Dimethylformamid und 7 ml Acetonitril wird bei -25° innerhalb 15 Minuten ein Gemisch von 1,8 g Oxalylchlorid in 4,5 ml Acetonitril zugetropft, das Gemisch anschliessend während 15 Minuten bei der gleichen Temperatur nachgerührt und dann 3,15 g 1,4-Dihydro-2,6-dimethyl-4-(3-nitrophenyl)-pyridin-3,5-dicarbonsäure-monomethylester zugegeben und die Suspension bei -15° während 20 Minuten gerührt, wobei eine rote Lösung entsteht. Hierauf wird ein Gemisch von 3,1 g 2-[4-(p-Fluorbenzoyl)-piperidin-1-yl]-1-phenyl-äthanol, 9,5 ml Pyridin und 2 ml Dimethylformaid bei -25° zugetropft und während 15 Stunden bei Raumtemperatur weiter gerührt. Die Reaktionslösung wird auf ein Gemisch aus wässriger 2N Soda-Lösung und Eis gegossen und mit Essigsäureäthylester erschöpfend extrahiert. Die organische Phase wird mit 2N Natronlauge, Wasser, 2N Salzsäure, Wasser und IN wässriger Natriumbicarbonatlösung gewaschen, über Natriumsulfat getrocknet und eingedampft. Das zurückbleibende Oel wird an einer Mitteldrucksäule mit einem Gemisch von Methylenchlorid/Methanol 9:1 chromatographiert, wobei man nach dem Aufarbeiten den 2,6-Dimethyl-4-(3-nitrophenyl)-1,4-dihydropyridin-3-carbonsäuremethylester-5-carbonsäure-[2-[4-(p-fluorbenzoyl)-piperidin-1-yl]-1-phenyl]-äthylester als Diastereomerengemisch in amorpher Form erhält. Die Verbindung wird durch folgende physikalische Kenndaten charakterisiert: 250 mHz FT-$^1$H-NMR ($CDCl_3$): 1.7 (m,4H,3.5-Piperidyl-$CH_2$); 2.5 (m,2H $>N-CH_2$); 2.4 (d,6H, DHP-$CH_3$); 2.6 (m,2H) und 2.9 (m,1H) und 3.05 (m,2H) 2.6-Piperidyl-$CH_2$ und 4H-Piperidyl); 3.7 (d,3H,$COOCH_3$); 5.2

(d,1H,4-Dihydropyridyl-H); 5.8 (s,1H-NH-); 5.9 (m,1H, Phenyl-CH-O-CO-); 6.9-8.2 (mehrere m, 14H, Phenyl-H).

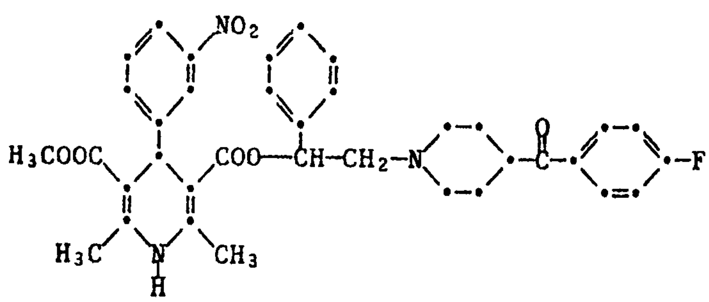

Das als Ausgangsmaterial verwendete 2-[4-(4-Fluorbenzoyl)-piperidin-1-yl]-1-phenyl-äthanol wird wie folgt hergestellt:

Ein Gemisch von 4-(4-Fluorbenzoyl)-piperidin, 2,9 g Styroloxid und 80 ml Tetrahydrofuran wird 15 Stunden am Rückfluss erhitzt, die Reaktionslösung anschliessend eingedampft und der Rückstand an einer Flashsäule in einem Gemisch von Methylenchlorid und Methanol 98:2 chromatographiert. Die nach dem Aufarbeiten erhaltene Verbindung wird als solche weiterverarbeitet.

Beispiel 2: Entsprechend der im Beispiel 1 beschriebenen Arbeitsweise erhält man durch Umsetzung von 3,37 g 1,4-Dihydro-2,6-dimethyl-4-(2-trifluormethyl-phenyl)-pyridin-3,5-dicarbonsäure-monomethylester mit 3,1 g 2-[4-(p-Fluorbenzoyl)-piperidin-1-yl]-1-phenyl-äthanol und nach dem dort beschriebenen Aufarbeiten den 2,6-Dimethyl-4-(2-trifluormethyl-phenyl)-1,4-dihydropyridin-3-carbonsäuremethylester-5-carbonsäure--[2-[4-(p-fluorbenzoyl)-piperidin-1-yl]-1-phenyl]-äthylester als Diastereomerengemisch in amorpher Form. Diese Verbindung wird durch folgende physikalische Kenndaten charakterisiert:

100 mHz $^1$H-NMR ($CDCl_3$): 2,35 (d,6H); 3,75 (d,3H); 5,2 (d,1H); 5,85 (s,1H): 5,9 (m,1H); 6,8-8,2 (m,13H).

Beispiel 3: Nach dem Verfahren in Beispiel 1 wird aus dem 5,0 g 1,4-Dihydro-2,6-dimethyl-4-(3-nitrophenyl)-pyridin-3,5-dicarbonsäure-monoethylester, 4,2 g 2-(4-Phenylpiperidin-1-yl)-1-phenyl-äthanol, 1,96 ml Oxalchlorid, 20 ml DMF, 28 ml Acetonitril und 10 ml Pyridin der 1,4 Dihydro-2,6-dimethyl-4-(3-nitrophenyl)-pyridin-3-carbonsäuremethylester-5-[2-(4-phenlypiperidin-1-yl)-1-phenyl]-äthylester hergestellt, der durch Flashchromatographie in einem Gemisch von Methylenchlorid/Methanol 95:5 gereinigt wird und in amorpher Form erhalten wird.

250 mHz FT-$^1$H-NMR ($CDCl_3$), TMS: 2,4 (d,6H,DHP-$CH_3$): 3.7 (d,3H,$COOCH_3$; 5.22 (s,1H,4-DHP-H; 5.7 (s,1H,NH): 5.9+6.0 (2m,1H,Phenyl-CH-O-CO); 6.95-8.15 (mehrere m,14H,Phenyl-H).

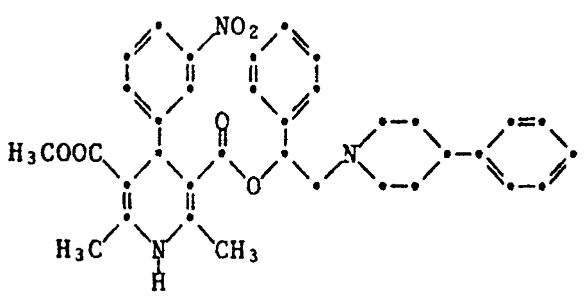

Beispiel 4: Analog den in der Beschreibung beschriebenen und dem in den Beispielen 1 bis 13 illustrierten Verfahren können, ausgehend von entsprechenden Ausgangsstoffen oder deren Salzen auch folgende Verbindungen der Formel I oder deren Salze, insbesondere deren pharmazeutisch verwendbaren, nicht-toxischen Säureadditionssalze hergestellt werden:

a) 2,6-Dimethyl-4-(3-nitrophenyl)-1,4-dihydropyridin-3-carbonsäuremethylester-5-carbonsäure-[6-[4-(p-fluorbenzoyl)-piperidin-1-yl]-1-phenyl]-n-hexylester; NMR-Daten 100 mHz, $^1$H-NMR -$CDCl_3$: 2,4(d,6H); 3,7(d,3H); 5,2(d,1H); 5,8(s,1H); 5,9(m,1H); 6,8-8,2(m,13H).

b) 2,6-Dimethyl-4-(3-nitrophenyl)-1,4-dihydropyridin-3-carbonsäuremethylester-[2-4-(p-chlorbenzoyl)-piperidin-1-yl]-1-phenyl]-äthylester; NMR-Daten 100 mHz, $^1$H-NMR($CDCl_3$: 2,3(d,6H); 3,75(d,3H); 5,2(d,1H); 5,8(s,1H); 5,9(m,1H); 6,8-8.2(m,13H).

c) 2,6-Dimethyl-4-(3-nitrophenyl)-1,4-dihydropyridin-3-carbonsäuremethylester-5-carbonsäure--[2-[4-(2-thenoyl)-piperidin-1-yl]-1-phenyl]-äthylester: NMR-Daten, 100 mHz, $^1$H-NMR($CDCl_3$): 2,25(d,6H); 3,8(d,3H); 5,1(d,1H); 5,7(s,1H); 5.8(m,1H); 7,1-8.2(m,12H).

d) 2,6-Dimethyl-4-(3-nitrophenyl)-1,4-dihydropyridin-3-carbonsäuremethylester-5-carbonsäure--[2-[4-(p-fluorphenyl)-piperidin-1-yl]-1-phenyl]-äthylester:NMR-Daten, 100 mHz, $^1$H-NMR($CDCl_3$): 2,35(d,6H); 3,7(d,3H); 5,1(d,1H); 5,6(s,1H); 5,9(m,1H); 6.8-8.2 (m,14H).

e) 2,6-Dimethyl-4-(3-nitrophenyl)-1,4-dihydropyridin-3-carbonsäuremethylester-5-carbonsäure-[2-[4-(p-fluorphenyl)-hydroxymethylen-piperidin-1-yl]-1-phenyl]-äthylester; NMR-Daten, 100 mHz, $^1$H-NMR($CDCl_3$): 2,4(d,6H); 3,65(d,3H); 5,15(d,1H); 5,65(s,1H); 5,85(m,1H); 6,8-8,2(m,14H).

f) 2,6-Dimethyl-4-(3-nitrophenyl)-1,4-dihydropyridin-3-carbonsäuremethylester-5-carbonsäure-[2-[4-(p-fluorbenzyl)-piperidin-1-yl]-1-phenyl]-äthylester; NMR-Daten, 100 mHz, $^1$H-NMR($CDCl_3$): 2,45 (d,6H); 3,7(d,3H); 5,1(d,1H); 5,7(s,1H); 5,9(m,1H); 6,8-8,2(m,14H).

g) 2,6-Dimethyl-4-(3-nitrophenyl)-1,4-dihydropyridin-3-carbonsäuremethylester-5-carbonsäure-[ 2-[4-(p-fluorbenzoyl)-piperidin-1-yl]-1-(p-methoxyphenyl)]-äthylester; NMR-Daten, 100 mHz, $^1$H-NMR($CDCl_3$): 2,35(d,6H); 3,75(d,3H): 5.15(d,1H); 5,6(s,1H); 5,75(m,1H); 6,8-8,2(m,12H).

h) 2,6-Dimethyl-4-(3-nitrophenyl)-1,4-dihydropyridin-3-carbonsäuremethylester-5-carbonsäure--[2-[4-(p-fluorbenzoyl)-piperidin-1-yl]-1-(p-cyanophenyl)]-äthylester; NMR-Daten, 100 mHz, $^1$H-NMR($CDCl_3$): 2,3(d,6H); 3,8(d,3H); 5,2(d.1H); 5,75(s,1H); 5,9(m,1H); 6,8-8,2(m,12H).

i) 2,6-Dimethyl-4-(3-nitrophenyl)-1,4-dihydropyridin-3-carbonsäuremethylester-5-carbonsäure-[2-[4-(p-fluorbenzoyl)-piperidin-1-yl]-1-(p-methylphenyl]-äthylester; NMR-Daten, 100 mHz, $^1$H-NMR($CDCl_3$): 2,35(d,6H); 3,7(d,3H); 5,1(d,1H); 5,65(s,1H); 5,85(m,1H); 6,8-8,2(m,12H).

j) 2,6-Dimethyl-4-(3-nitrophenyl)-1,4-dihydropyridin-3-carbonsäuremethylester-5-carbonsäure-[2-[4-(p-fluorbenzoyl)-piperidin-1-yl]-1-(p-chlorphenyl)]-äthylester; NMR-Daten, 100 mHz, $^1$H-NMR($CDCl_3$): 2,45(d,6H); 3,85(d,3H); 5,05(d,1H); 5,8(s,1H); 5,85(m,1H); 6,8-8,2(m,12H).

k) 2,6-Dimethyl-4-(3-nitrophenyl)-1,4-dihydropyridin-3-carbonsäuremethylester-5-carbonsäure-[2-[4-(p-fluorbenzoyl)-piperidin-1-yl]-1-(p-trifluormethylphenyl)]-äthylester; NMR-Daten, 100 mHz, $^1$H-NMR($CDCl_3$: 2,45(d,6H); 3,7(d,3H); 5,15(d,1H); 5,6(s,1H); 5,75 (m,1H); 6,8-8,2(m,12H).

l) 2,6-Dimethyl-4-(3-nitrophenyl)-1,4-dihydropyridin-3-carbonsäuremethylester-5-carbonsäure-[2-[4-(p-fluorbenzoyl)-piperidin--1-yl]-1-phenyl]-propylester; NMR-Daten, 100 mHz, $^1$H-NMR($CDCl_3$): 2,4(d,6H); 3,65(d,3H); 5,2(d,1H); 5,75(s,1H); 5,8(m,1H); 6,8-8,2 (m,13H).

m) 2,6-Dimethyl-4-(3-nitrophenyl)-1,4-dihydropyridin-3-carbonsäuremethylester-5-carbonsäure--[4-[4-(p-fluorbenzoyl)-piperidin-1-yl]-1-phenyl]-butylester; NMR-Daten, 100 mHz, $^1$H-NMR($CDCl_3$): 2,35(d,6H); 3,8(d,3H); 5,1(d,1H); 5,6(s,1H); 5,9(m,1H); 6,8-8.2 (m,13H).

n) 2,6-Dimethyl-4-(3-nitrophenyl)-1,4-dihydropyridin-3-carbonsäuremethylester-5-carbonsäure--[2-[4-(p-fluorbenzoyl)-piperidin-1-yl]-2-phenyl]-äthylester; NMR-Daten, 100 mHz, $^1$H-NMR($CDCl_3$): 2,3(d,6H); 3,65(d,3H); 5,15(s,1H); 5,7(m,1H); 6,8-8,2(m,13H).

o) 2,6-Dimethyl-4-(3-nitrophenyl)-1,4-dihydropyridin-3-carbonsäuremethylester-5-carbonsäure--[3-[4-(p-fluorbenzoyl)-piperidin-1-yl]-3-phenyl]-propylester; NMR-Daten, 100 mHz, $^1$H-NMR($CDCl_3$): 2,45(d,6H); 3,7(d,3H); 5,2(d,1H); 5,7(s,1H); 5.85(m,1H); 6,8-8.2 (m,13H).

p) 2,6-Dimethyl-4-(3-trifluormethylphenyl)-1,4-dihydropyridin-3-carbonsäuremethylester-5-carbonsäure-[2-[4-(p-fluorbenzoyl)-piperidin-1-yl]-1-phenyl]-äthylester; NMR-Daten, 100 mHz, $^1$H-NMR($CDCl_3$): 2,4(d,6H); 3,8(d,3H); 5,3(d,1H); 5,8(s,1H): 5.95(m,1H); 6,8-8,2(m.1H).

q) 2,6-Dimethyl-4-(2,3-dichlorphenyl)-1.4-dihydropyridin-3-carbonsäuremethylester-5-carbonsäure--[2-[4-(p-fluorbenzoyl)-piperidin-1-yl]-1-phenyl]-äthylester; NMR-Daten, 100 mHz, $^1$H-NMR($CDCl_3$): 2.45(d,6H); 3,7(d,3H); 5,25(d,1H); 5,8(s.1H); 5,85(m,1H); 6,8-8,2(m,12H).

r) 2,6-Dimethyl-4-(2-nitrophenyl)-1.4-dihydropyridin-3-carbonsäuremethylester-5-carbonsäure-[2-[4-(p-fluorbenzoyl)-piperidin-1-yl]-1-phenyl ]-äthylester; NMR-Daten, 100 mHz, $^1$H-NMR($CDCl_3$): 2,35 (d,6H); 3,75(d,3H); 5,2(d,1H); 5,8(s,1H); 5.9(m,1H); 6,8-8,2(m,13H).

Beispiel 5: Tabletten enthaltend 20 mg an aktiver Substanz werden in folgender Zusammensetzung in üblicher Weise hergestellt:

Zusammensetzung:

| | |
|---|---|
| z.B. 2,6-Dimethyl-4-(3-nitrophenyl)-1,4-dihydropyridin-3-carbonsäuremethylester-5-carbonsäure-[2-[4-(p-fluorbenzoyl)-piperidin-1-yl]-1-phenyl]-äthylester | 20 mg |
| Weizenstärke | 60 mg |
| Milchzucker | 50 mg |
| Kolloidale Kieselsäure | 5 mg |
| Talk | 9 mg |
| Magnesiumstearat | 1 mg |
| | 145 mg |

Herstellung:

Der Wirkstoff wird mit einem Teil der Weizenstärke, mit Milchzucker und kolloidaler Kieselsäure gemischt und die Mischung durch eine Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastiche Masse entstanden ist.

Die plastische Masse wird durch ein Sieb von ca. 3 mm Maschenweite gedrückt, getrocknet und das erhaltene trockene Granulat nochmals durch ein Sieb getrieben. Darauf werden die restliche Weizenstärke, Talk und Magnesiumstearat zugemischt und die Mischung zu Tabletten von 145 mg Gewicht mit Bruchkerbe verpresst.

Beispiel 6: Tabletten enthaltend 1 mg an aktiver Substanz werden in folgender Zusammensetzung in üblicher Weise hergestellt:

Zusammensetzung:

| | |
|---|---|
| z.B. 2,6-Dimethyl-4-(3-nitrophenyl)-1,4-dihydropyridin-3-carbonsäuremethylester-5-carbonsäure-[2-[4-(p-fluorbenzoyl)-piperidin-1-yl]-1-phenyl]-äthylester | 1 mg |
| Weizenstärke | 60 mg |
| Milchzucker | 50 mg |
| Kolloidale Kieselsäure | 5 mg |
| Talk | 9 mg |
| Magnesiumstearat | 1 mg |
| | 126 mg |

Herstellung:

Der Wirkstoff wird mit einem Teil der Weizenstärke, mit Milchzucker und kolloidaler Kieselsäure gemischt und die Mischung durch ein Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastische Masse entstanden ist.

Die plastische Masse wird durch ein Sieb von ca. 3 mm Maschenweite gedrückt, getrocknet und das erhaltene trockene Granulat nochmals durch ein Sieb getrieben. Darauf werden die restliche Weizenstärke, Talk und Magnesiumstearat zugemischt und die Mischung zu Tabletten von 126 g Gewicht mit Bruchkerbe verpresst.

Beispiel 7: Kapseln enthaltend 10 mg an aktiver Substanz werden wie folgt auf übliche Weise hergestellt:

Zusammensetzung:

| | |
|---|---|
| z.B. 2,6-Dimethyl-4-(3-nitrophenyl)-1,4-dihydro-pyridin-3-carbonsäuremethylester-5-carbon-säure-[2-[4-(p-fluorbenzoyl)-piperidin-1-yl]-1-phenyl]-äthylester | 2500 mg |
| Talkum | 200 mg |
| Kolloidale Kieselsäure | 50 mg |

Herstellung:

Die aktive Substanz wird mit Talkum und kolloidaler Kieselsäure innig gemischt, das Gemisch durch ein Sieb mit 0,5 mm Maschenweite getrieben und dieses in Portionen von jeweils 11 mg in Hartgelatinekapseln geeigneter Grösse abgefüllt.

Beispiel 8: Anstelle der in den Beispielen 4 bis 6 als aktive Substanz verwendeten Verbindungen können auch die in den Beispielen 2 bis 4 genannten Verbindungen der Formel I oder deren pharmazeutisch annehmbaren, nicht-toxischen Säureadditionssalze als Wirkstoffe in Tabletten, Dragées, Kapseln etc. verwendet werden.

## Patentansprüche

1.Verbindungen der Formel

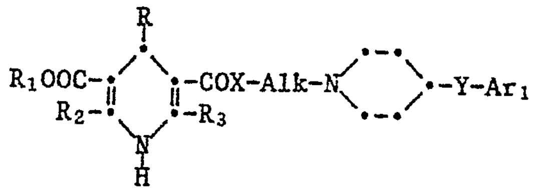

(I),

worin R einen carbocyclischen oder heterocyclischen Arylrest bedeutet, $R_1$ Niederalkyl darstellt, eine der Gruppen $R_2$ und $R_3$ für Niederalkyl und die andere für Niederalkyl, Cyano oder Amino steht, X Sauerstoff oder die Gruppe -NH- darstellt, und Alk durch ein carbocyclisches Aryl substituiertes Alkylen bedeutet, das die Gruppe X vom Ringstickstoffatom durch mindestens zwei Kohlenstoffatome trennt, Y für Alkylen oder die Gruppen -CH(OH)- oder -C(=O)- steht oder eine einfache Bindung bedeutet, und $Ar_1$ einen monocyclischen Aryl- oder Heteroarylrest darstellt, und ihre Salze.

2. Verbindungen gemäss Anspruch 1 der Formel I, in welchen R für einen mono- oder bicyclischen, carbocyclischen Arylrest oder für einen fünf- oder sechsgliedrigen, ein bis und mit vier Ringstickstoffatome, ein Ringsauerstoff- oder Ringschwefelatom, oder ein oder zwei Ringstickstoffatome zusammen mit einem Ringsauerstoff- oder einem Ringschwefelatom als Ringglieder enthaltenden, monocyclischen Heteroarylrest steht, der über ein Ringkohlenstoffatom mit dem Kohlenstoffatom der 4-Stellung des 1,4-Dihydropyridinrings verbunden ist, und der gegebenenfalls einen ankondensierten Benzoring enthält, und insbesondere Phenyl, Naphthyl, Pyrryl, Pyrazolyl, Imidazolyl, Triazolyl, Tetrazolyl, Furyl, Thienyl, Isoxazolyl, Oxazolyl, Oxadiazolyl, Isothiazolyl, Thiazolyl, Thiadiazolyl, Pyridyl, Pyridazinyl, Pyrimidinyl, Pyrazinyl, Triazinyl, Indolyl, Isoindolyl, Benzimidazolyl, Benzofuranyl, Benzofurazanyl, Benzothienyl, Benzthiazolyl, 2,1,3-Benzthiadiazolyl, Chinolinyl oder Isochinolinyl bedeutet, wobei in diesen Resten Ringkohlenstoffatome gegebenenfalls durch Niederalkyl, Niederalkenyl, Niederalkinyl, Niederalkylen, Cycloalkyl, Phenyl und/oder Phenylniederalkyl, wobei Niederalkyl, Phenyl oder Phenylniederalkyl gegebenenfalls Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkyl-carbamoyl und/oder Cyan, und cyclische Reste auch Niederalkyl, das seinerseits wie angegeben substituiert sein kann, als Substituenten enthalten können, und/oder durch Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Nitro, Amino, N-Niederalkyl-amino, N,N-Diniederalkyl-amino, N-Niederalkyl-N-phenylniederalkyl-amino, Niederalkylenamino, Oxaniederalkylenamino, Thianiederalkylenamino und/oder Azaniederalkylenamino, worin das Azastickstoffatom durch Niederalkyl, Phenyl oder Phenylniederalkyl substituiert sein kann, wobei diese Reste Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkyl-carbamoyl und/oder Cyan, die cyclischen Reste auch Niederalkyl als Substituenten enthalten können, und/oder durch Niederalkanoylamino, Azido, Niederalkanoyl, Carboxy, Niederalkoxycarbonyl, Carbamoyl, N-Niederalkyl-carbamoyl, N,N-Diniederalkyl-carbamoyl, Cyan, Sulfo, Aminosulfonyl, N-Niederalkylaminosulfonyl, N,N-Diniederalkylaminosulfonyl, Niederalkylthio, Halogenniederalkylthio, Niederalkylsulfinyl und/oder Niederalkylsulfonyl, und/oder Ringstickstoffatome gegebenenfalls durch Niederalkyl, das als Substituenten gegebenenfalls Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkylcarbamoyl, N,N-Diniederalkyl-carbamoyl oder Cyan enthalten kann, oder durch Hydroxy oder Oxido substituiert sein können, $R_1$ Niederalkyl ist, einer der Reste $R_2$ und $R_3$ für Niederalkyl und der andere für Niederalkyl, Cyano oder Amino steht, X Sauerstoff oder die Gruppe -NH- bedeutet, und Alk durch ein carbocyclisches Aryl, z.B. durch gegebenenfalls, etwa wie nachfolgend für carbocyclisches Aryl $Ar_1$ angegeben, substituiertes Phenyl oder Naphthyl. substituiertes Alkylen darstellt, das die Gruppe X vom Ringstickstoffatom durch 2 bis 8 Kohlenstoffatome trennt, Y für Alkylen mit 1 bis 6 Kohlenstoffatomen oder für die Gruppen -CH(OH)- oder -C(=O)- steht oder eine einfache Bindung bedeutet, und $Ar_1$ einen monocyclischen carbocyclischen Aryl- oder Heteroarylrest darstellt, und insbesondere Phenyl, Naphthyl, Pyrryl, Furyl, Thienyl oder Pyridyl bedeutet, wobei in diesen Resten Ringkohlenstoffatome gegebenenfalls durch Niederalkyl, Niederalkenyl, Niederalkinyl, Niederalkylen, Cycloalkyl, Phenyl und/oder Phenylniederalkyl substituiert sind, wobei Niederalkyl. Phenyl oder Phenylniederalkyl als Substituenten Hydroxy,

Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkyl-carbamoyl und/oder Cyan, und cyclische Reste auch Niederalkyl, das seinerseits wie angegeben substituiert sein kann, enthalten können, und/oder durch Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Nitro, Amino, N-Niederalkyl-amino, N,N-Diniederalkylamino, N-Niederalkyl-N-phenylniederalkyl-amino, Niederalkylenamino, Oxoniederalkylenamino, Thianiederalkylenamino und/oder Azaniederalkylenamino, worin das Azastickstoffatom durch Niederalkyl, Phenyl oder Phenylniederalkyl substituiert sein kann, wobei diese Reste Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N,-Diniederalkylcarbamoyl und/oder Cyan, die cyclischen Reste auch Niederalkyl als Substituenten enthalten können, und/oder durch Niederalkanoylamino, Azido, Niederalkanoyl, Carboxy, Niederalkoxycarbonyl, Carbamoyl, N-Niederalkyl-carbamoyl, N,N-Diniederalkyl-carbamoyl, Cyan, Sulfo, Aminosulfonyl, N-Niederalkylaminosulfonyl, N,N-Diniederalkylaminosulfonyl, Niederalkylthio, Niederalkylsulfinyl und/oder Niederalkylsulfonyl, und/oder Ringstickstoffatome gegebenenfalls durch Niederalkyl, das als Substituenten gegebenenfalls Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkylcarbamoyl, N,N-Diniederalkyl-carbamoyl oder Cyan als Substituenten enthalten kann, oder durch Hydroxy oder Oxido substituiert sein können, und ihre Salze.

3. Verbindungen gemäss Anspruch 1 der Formel I, worin R und $Ar_1$ jeweils für Phenyl oder Naphthyl stehen, das gegebenenfalls durch Niederalkyl, Phenyl und/oder Phenylniederalkyl, wobei solche Reste ihrerseits Hydroxy, Niederalkoxy, Halogenniederalkoxy, Niederalkylendioxy, Halogen, Carboxy, Niederalkoxycarbonyl und/oder Cyan, die cyclischen Reste auch Niederalkyl als Substituenten enthalten können, und/oder durch Hydroxy, Niederalkoxy, Halogenniederalkoxy, Niederalkenyloxy, Halogenniederalkenyloxy, Niederalkylendioxy, Halogen, Nitro, Amino, N-Niederalkylamino, N,N-Diniederalkylamino, Niederalkanoylamino, Carboxy, Niederalkoxycarbonyl, Carbamoyl, Cyan, Sulfo, Aminosulfonyl, N-Niederalkylaminosulfonyl, N,N-Diniederalkylaminosulfonyl, Niederalkylthio, Niederalkylsulfinyl und/oder Niederalkylsulfonyl substituiert ist, oder R und $Ar_1$ jeweils für über ein Ringkohlenstoffatom gebundenes Pyrryl, Furyl, Thienyl, Pyridyl, 1-Oxido-pyridyl oder Imidazolyl, R auch für Benzofurazanyl steht, wobei solche Reste gegebenenfalls, wie für einen Phenyl- oder Naphthylrest R bzw. $Ar_1$ angegeben, substituiert sind, und insbesondere Niederalkyl, Niederalkoxy, Halogen und/oder gegebenenfalls durch Niederalkyl, Niederalkoxy, Halogen und/oder Nitro substituiertes Phenyl als Substituenten enthalten können, $R_1$ Niederalkyl ist, eine der Gruppen $R_2$ und $R_3$ Niederalkyl ist und die andere Niederalkyl oder Cyano darstellt, X Sauerstoff oder die Gruppe -NH- bedeutet, und Alk durch ein gegebenenfalls durch Niederalkyl, Niederalkoxy, Hydroxy, Halogenniederalkyl, Halogen, Cyan und/oder Nitro substituiertes Phenyl oder Naphthyl substi tuiertes Alkylen mit 2 bis 6 Kohlenstoffatomen steht, das die Gruppe X vom Ringstickstoffatom durch 2 bis 6 Kohlenstoffatome trennt und Y Alkylen mit 1 bis 4 Kohlenstoffatomen oder die Gruppen -CH(OH)-oder -C(=O) bedeutet, und ihre Salze.

4. Verbindung gemäss Anspruch 1 der Formel I, worin R und $Ar_1$ jeweils für Phenyl stehen, das gegebenenfalls durch Niederalkyl, Hydroxy, Niederalkoxy, Halogenniederalkoxy, Halogenniederalkenyloxy, Niederalkylendioxy, Halogen, Trifluormethyl, Nitro, Niederalkanoylamino, gegebenenfalls durch gegebenenfalls verestertes oder veräthertes Hydroxy substituiertes Phenyl oder Phenylniederalkyl, und/oder Cyan substituiert ist, wobei ein Phenylrest R bzw. $Ar_1$ einen oder mehrere, gleiche oder verschiedene Substituenten aufweisen kann, oder R und $Ar_1$ jeweils Pyridyl, Furyl, 1-Oxido-pyridyl, oder Thienyl, R auch Benzofurazanyl, z.B. 4-Benzofurazanyl, bedeutet, das durch Niederalkyl oder Halogen substituiert sein kann, $R_1$, $R_2$ und $R_3$ jeweils Niederalkyl bedeuten, X für die Gruppe -NH-, insbesondere jedoch für Sauerstoff steht, Alk durch ein gegebenenfalls Niederalkyl, Niederalkoxy, Hydroxy, Halogenniederalkyl, Halogen, Cyan und/oder Nitro enthaltendes Phenyl substituiertes Alkylen mit 2 bis 4 Kohlenstoffatomen bedeutet, wobei der gegebenenfalls substituierte Phenylrest vorzugsweise an das an die Gruppe X benachbarte Kohlenstoffatome gebunden ist, und Alk die Gruppe X vom Ringstickstoffatom durch 2 bis 4 Kohlenstoffatome trennt, Y für Alkylen mit 1 bis 3 Kohlenstoffatomen oder die Gruppen -CH(OH)- oder insbesondere -C(=O)- steht, und ihre Salze.

5. Verbindungen gemäss Anspruch 1 der Formel I, worin R für unsubstituiertes oder vorzugsweise durch Niederalkyl, z.B. Methyl, Niederalkoxy, z.B. Methoxy, Halogenniederalkoxy, z.B. Difluormethoxy, oder Halogenniederalkenyloxy, worin Halogen eine Atomnummer bis und mit 35 hat und insbesondere Fluor oder Chlor ist, z.B. 1,1-Difluorvinyloxy, Halogen mit einer Atomnummer bis und mit 35, insbesondere Chlor sowie Fluor, Trifluormethyl, Nitro und/oder Cyan mono- oder di-substituiertes Phenyl steht, wobei Substituenten die 2- und/oder 3-Stellung einnehmen, $R_1$ Niederalkyl z.B.Methyl oder Aethyl, und $R_2$ und $R_3$ Niederalkyl, insbesondere Methyl, bedeuten, X für die Gruppe -NH-, insbesondere jedoch für Sauerstoff steht, Alk durch ein durch Niederalkyl, Niederalkoxy, Hydroxy, Halogenniederalkyl, Halogen, Cyan und/oder Nitro substituiertes, insbesondere aber durch ein unsubstituiertes Phenyl substituiertes Alkylen mit 1 bis 3 Kohlenstoffatomen darstellt, wobei der gegebenenfalls substituierte Phenylrest an das der Gruppe X benachbarte Kohlenstoffatom gebunden ist. und Alk die Gruppe X vom Ringstickstoffatom

durch 2 bis 3 Kohelnstoffatome trennt, Y für Alkylen mit 1 bis 3 Kohlenstoffatomen oder insbesondere die Gruppe -C(=O)- steht, und $Ar_1$ gegebenenfalls durch Halogen mit einer Atomnummer bis und mit 35, z.B. Fluor oder Chlor, substituiertes Phenyl oder Pyridyl bedeutet, und ihre Salze.

6. Verbindungen gemäss Anspruch 1 der Formel I, worin R durch Halogenniederalkoxy, insbesondere mit einer Atomnummer bis und mit 35 und mit bis 4 C-Atomen, wie Difluormethoxy, Niederalkylendioxy, insbesondere mit bis und mit 3 C-Atomen, wie Methylendioxy, Halogen, insbesondere mit einer Atomnummer bis und mit 35, wie Chlor, Trifluormethyl oder Nitro mono- oder disubstituiertes Phenyl, wobei Substituenten die 2- und/oder 3-Stellung einnehmen, Benzofurazanyl, insbesondere 4-Benzofurazanyl, oder 1-Oxido-pyridyl, wie 1-Oxido-3-pyridyl, bedeutet, $R_1$, $R_2$ und $R_3$ unabhängig voneinander Niederalkyl, insbesondere mit bis und mit 4 C-Atomen, wie Methyl, bedeuten, X für Sauerstoff steht, Alk durch ein gegebenenfalls Niederalkyl, Niederalkoxy, jeweils insbesondere mit bis und mit 4 C-Atomen, wie Methyl oder Methoxy, Halogenniederalkyl, insbesondere mit einer Atomnummer bis und mit 35 und mit bis und mit 4 C-Atomen, wie Trifluormethyl, Halogen, insbesondere mit einer Atomnummer bis und mit 35, wie Chlor, oder Cyan substituiertes Phenyl enthaltendes Alkylen mit 2 bis 4 C-Atomen bedeutet, wobei Alk die Gruppe X vom Ringstickstoffatom durch 2 bis 4 C-Atome trennt, Y für Alkylen mit 1 bis 3 C-Atomen, die Gruppen -CH(OH)- oder insbesondere -C(=O)- oder eine Bindung steht, und $Ar_1$ gegebenenfalls durch Halogen, insbe sondere mit einer Atomnummer bis und mit 35, wie Chlor, substituiertes Phenyl oder Thienyl, wie 2-Thienyl, bedeutet, und ihre Salze.

7. Verbindungen gemäss Anspruch 1 der Formel I, worin R durch Halogen mit einer Atomnummer bis und mit 35, Chlor, Trifluormethyl oder Nitro mono- oder disubstituiertes Phenyl steht, wobei Substituenten bevorzugt die 2- oder 3-Stellung einnehmen, $R_1$, $R_2$ und $R_3$ unabhängig voneinander Niederalkyl mit bis und mit 4 C-Atomen, wie Methyl oder Aethyl, bedeuten, X für Sauerstoff steht, Alk ein durch Niederalkyl, Niederalkoxy, jeweils mit bis und mit 4 C-Atomen, wie Methyl oder Methoxy, Halogenniederalkyl mit einer Atomnummer bis und mit 35 und mit bis und mit 4 C-Atomen, wie Trifluormethyl, Halogen mit einer Atomnummer bis und mit 35, wie Chlor, oder Cyan substituiertes oder unsubstituiertes Phenyl substituiertes Alkylen mit 2 bis und mit 6 C-Atomen, bedeutet, wobei der gegebenenfalls substituierte Phenylrest vorzugsweise an das an die Gruppe X benachbarte C-Atom gebunden ist und die Gruppe X vom Ringstickstoffatom durch mindestens zwei C-Atome getrennt wird, Y für Alkylen mit 1 bis 3 C-Atomen, die Gruppen -CH(OH)- oder -C(=O)-, ferner eine Bindung steht, und $Ar_1$ unsubstituiertes oder durch Halogen mit einer Atomnummer bis und mit 35, wie Fluor, substituiertes Phenyl oder Thienyl, wie 2-Thienyl, bedeutet, und ihre Salze.

8. Verbindungen gemäss Anspruch 1 der Formel I, worin R für 2-, 3-Nitrophenyl, 2-, 3-Trifluormethylphenyl oder 2,3-Dichlorphenyl steht, $R_1$, $R_2$ und $R_3$ unabhängig voneinander jeweils Niederalkyl mit bis und mit 4 C-Atomen, insbesondere Methyl, bedeuten, X für Sauerstoff steht, Alk ein durch Niederalkyl mit bis und mit 4 C-Atomen, wie Methyl, Niederalkoxy mit bis und mit 4 C-Atomen, wie Methoxy, Halogenniederalkyl mit einer Atomnummer bis und mit 35 und mit bis und mit 4 C-Atomen, wie Trifluormethyl, Halogen mit einer Atomnummer bis und mit 35, wie Chlor, oder Cyano monosubstituiertes oder unsubstituiertes Phenyl substituiertes Alkylen mit 2 bis und mit 6 C-Atomen bedeutet und wobei die Gruppen X vom Ringstickstoffatom durch mindestens zwei C-Atome getrennt wird, Y für Methylen, Hydroxymethylen, die Gruppe -C(=O)- oder eine Bindung steht, und $Ar_1$ unsubstituiertes oder durch Halogen mit einer Atomnummer bis und mit 35, wie Fluor, monosubstituiertes Phenyl oder Thienyl, wie 2-Thienyl, bedeutet, und ihre Salze.

9. Verbindungen gemäss Anspruch 1 der Formel I, worin R für durch Halogen mit einer Atomnummer bis und mit 35, besonders Fluor oder Chlor, mono oder disubstituiertes oder durch Trifluormethyl, Nitro oder Cyan monosubstituiertes Phenyl steht, wobei Substituenten die 2- und/oder 3-Stellung einnehmen, $R_1$ Niederalkyl, z.B. Methyl oder Aethyl, und $R_2$ und $R_3$ jeweils Niederalkyl, in erster Linie Methyl, bedeuten, X für Sauerstoff steht, Alk durch ein durch Niederalkyl, z.B. Methyl, Niederalkoxy, z.B. Methoxy, Trifluormethyl, Cyan und/oder Nitro substituiertes, insbesondere aber durch ein unsubstituiertes, Phenyl substituiertes Alkylen mit 2 bis 3 Kohlenstoffatomen darstellt, wobei der gegebenenfalls substituierte Phenylrest an das der Gruppe X benachbarte Kohlenstoffatome gebunden ist, und Alk die Gruppe X vom Ringstickstoffatom durch 2 bis 3 Kohlenstoffatome trennt, Y für Alkylen mit 1 bis 2 Kohlenstoffatomen, oder insbesondere die Gruppe -C(=O)- steht, und $Ar_1$ gegebenenfalls, insbesondere in 4-Stellung, durch Halogen mit einer Atomnummer bis und mit 35, z.B. Fluor, substituiertes Phenyl bedeutet, und ihre Salze.

10. Verbindungen gemäss Anspruch 1 der Formel I, worin R 3-Nitrophenyl oder 2-Trifluormethylphenyl bedeutet, $R_1$ Methyl oder Aethyl bedeutet, $R_2$ und $R_3$ Methyl bedeuten, X für Sauerstoff steht, Alk 1-Phenyläthyl bedeutet, Y für die Gruppe -C(=O)- steht und $Ar_1$ gegebenenfalls in 4-Stellung durch Fluor substituiertes Phenyl bedeutet, und ihre Salze.

11. 2,6-Dimethyl-4-(3-nitrophenyl)-1,4-dihydropyridin-3-carbonsäuremethylester-5-carbonsäure-[2-[4-(p-fluorbenzoyl)-piperidin-1-yl]-1-phenyl]-äthylester oder ein Salz davon.

12. 2,6-Dimethyl-4-(2-trifluormethyl-phenyl)-1,4-dihydropyridin3-carbonsäuremethylester-5-carbonsäure-[2-[4-(p-fluorbenzoyl)-piperidin-1-yl]-1-phenyl]-äthylester oder ein Salz davon.

13. 1,4-Dihydro-2,6-Dimethyl-4-(3-nitrophenyl)-pyridin-3-carbonsäuremethylester-5-[2-(4-phenylpiperidin-1-yl)-1-phenyl]-äthylester oder ein Salz davon.

14. 2,6-Dimethyl-4-(3-nitrophenyl)-1,4-dihydropyridin-3-carbonsäuremethylester-5-carbonsäure-[6-[4-(p-fluorbenzoyl)-piperidin-1-yl]-1-phenyl]-n-hexylester. 2,6-Dimethyl-4-(3-nitrophenyl)-1,4-dihydropyridin-3-carbonsäuremethylester-[2-[4-(p-chlorbenzoyl)-piperidin-1-yl]-1-phenyl]-äthylester. 2,6-Dimethyl-4-(3-nitrophenyl)-1,4-dihydropyridin-3-carbonsäuremethylester-5-carbonsäure-[2-[4-(2-thenoyl)-piperidin-1-yl]-1-phenyl]-äthylester. 2,6-Dimethyl-4-(3-nitrophenyl)-1,4-dihydropyridin-3-carbonsäuremethylester-5-carbonsäure-[2-[4-(p-fluorphenyl)-piperidin-1-yl]-1-phenyl]-äthylester, 2,6-Dimethyl-4-(3-nitrophenyl)-1,4-dihydropyridin-3-carbonsäuremethylester-5-carbonsäure-[ 2-[4-(p-fluorphenyl)-hydroxymethylen-piperidin-1-yl]-1-phenyl]-äthylester, 2,6-Dimethyl-4-(3-nitrophenyl)-1,4-dihydropyridin-3-carbonsäuremethylester-5-carbonsäure-[2-[4-(p-fluorbenzoyl)-piperidin-1-yl]-1-phenyl]-äthylester, 2,6-Dimethyl-4-(3-nitrophenyl)-1,4-dihydropyridin-3-carbonsäuremethylester-5-carbonsäure-[2-[4-(p-fluorbenzoyl)-piperidin-1-yl]-1-(p-methoxyphenyl)]-äthylester, 2,6-Dimethyl-4-(3-nitrophenyl)-1,4-dihydropyridin-3-carbonsäuremethylester-5-carbonsäure-[2-[4-(p-fluorbenzoyl)-piperidin-1-yl]-1-(p-cyanophenyl)]-äthylester, 2,6-Dimethyl-4-(3-nitrophenyl)-1,4-dihydropyridin-3-carbonsäuremethylester-5-carbonsäure-[2-[4-(p-fluorbenzoyl)-piperidin-1-yl]-1-(p-methylphenyl]-äthylester, 2,6-Dimethyl-4-(3-nitrophenyl)-1,4-dihydropyridin-3-carbonsäuremethylester-5-carbonsäure-[2-[4-(p-fluorbenzoyl)-piperidin--1-yl]-1-(p-chlorphenyl)]-äthylester, 2,6-Dimethyl-4-(3-nitrophenyl)-1,4-dihydropyridin-3-carbonsäuremethylester-5-carbonsäure-[2-[4-(p-fluorbenzoyl)-piperidin-1-yl]-1-(p-trifluormethylphenyl)]-äthyl ester, 2,6-Dimethyl-4-(3-nitrophenyl)-1,4-dihydropyridin-3-carbonsäuremethylester-5-carbonsäure-[3-[4-(p-fluorbenzoyl)-piperidin-1-yl]-1-phenyl]-propylester, 2,6-Dimethyl-4-(3-nitrophenyl)-1,4-dihydropyridin-3-carbonsäuremethylester-5-carbonsäure-[4-[4-(p-fluorbenzoyl)-piperidin-1-yl]-1-phenyl]-butylester, 2,6-Dimethyl-4-(3-nitrophenyl)-1,4-dihydropyridin-3-carbonsäuremethylester-5-carbonsäure-[2-[4-(p-fluorbenzoyl)-piperidin-1-yl]-2-phenyl]-äthylester, 2,6-Dimethyl-4-(3-nitrophenyl)-1,4-dihydropyridin-3-carbonsäuremethylester-5-carbonsäure-[3-[4-(p-fluorbenzoyl)-piperidin-1-yl]-3-phenyl]-propylester, 2,6-Dimethyl-4-(3-trifluormethylphenyl)-1,4-dihydropyridin-3-carbonsäuremethylester-5-carbonsäure-[2-[4-(p-fluorbenzoyl)-piperidin-1-yl]-1-phenyl]-äthylester, 2,6-Dimethyl-4-(2,3-dichlorphenyl)-1,4-dihydropyridin-3-carbonsäuremethylester-5-carbonsäure-[2-[4-(p-fluorbenzoyl)-piperidin-1-yl]-1-phenyl]-äthylester, 2,6-Dimethyl-4-(2-nitrophenyl)-1,4-dihydropyridin-3-carbonsäuremethylester-5-carbonsäure-[2-[4-(p-fluorbenzoyl)-piperidin-1-yl]-1-phenyl ]-äthylester und ein Salz davon.

15. Verbindungen gemäss einem der Ansprüche 1-14 oder ein pharmazeutisch verwendbares, nicht-toxiches Säureadditionssalz davon zur Anwendung in einem Verfahren zur therapeutischen Behandlung des menschlichen oder tierischen Körpers.

16. Verbindungen gemäss einem der Ansprüche 1-14 oder ein pharmazeutisch verwendbares, nicht-toxisches Säureadditionssalz davon zur Verwendung als Coronardilatatoren und Antihypertensiva.

17. Pharmazeutische Präparate enthaltend eine Verbindung gemäss einem der Ansprüche 1-16 in freier Form in oder in Form eines pharmazeutisch verwendbaren. nicht-toxischen Salzes.

18. Verwendung von Verbindungen der Formel I in freier Form oder in Form eines pharmazeutisch verwendbaren, nicht-toxischen Salzes davon zur Herstellung von Coronardilatatoren und Antihypertensiva.

19. Verfahren zur Herstellung von Verbindungen der Formel I gemäss Anspruch 1, dadurch gekennzeichnet, dass man a) eine Verbindung der Formel

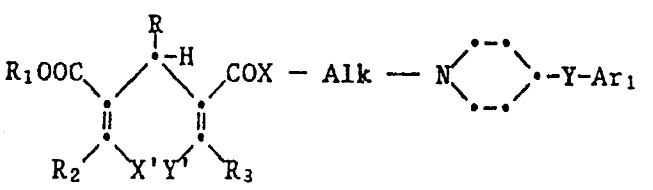

(II),

worin einer der Reste X' und Y' für die Gruppe der Formel $-NH_2$ steht und der andere Hydroxy oder die Gruppe der Formel $-NH_2$ bedeutet, oder ein Tautomeres davon oder ein entsprechendes Tautomerengemisch ringschliesst, oder

b) eine Verbindung der Formel R-CHO (III) oder ein reaktionsfähiges funktionelles Derivat davon mit einer Verbindung der Formel

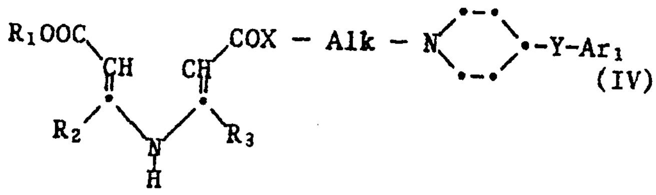

(IV)

oder einem Tautomeren davon oder einem entsprechenden Tautomerengemisch umsetzt, oder

c) in einer Verbindung der Formel

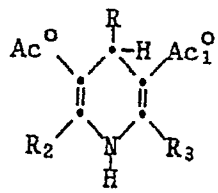

(V),

in welcher einer der Reste $Ac^o$ und $Ac_1^o$ eine in die Gruppe $-COOR_1$ bzw. in den Rest der Formel

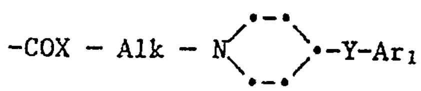

(Va)

überführbare Gruppe bedeutet, und der andere die Gruppe $-COOR_1$ oder die Gruppe der Formel Va oder einen in die Gruppe $-COOR_1$ bzw. die Gruppe der Formel Va überführbaren Rest bedeutet, den Rest $Ac^o$ in die Gruppe $-COOR_1$ und/oder den Rest $Ac_1^o$ in einen Rest der Formel Va überführt, oder

d) eine Verbindung der Formel

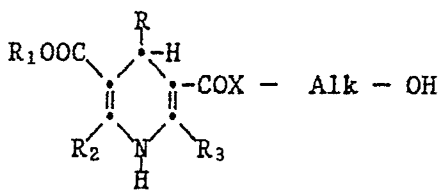

(VI)

oder einen reaktionsfähigen Ester davon, mit einer Verbindung der Formel

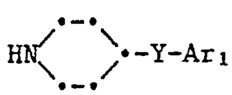

(VIa)

umsetzt, oder

e) eine Verbindung der Formel

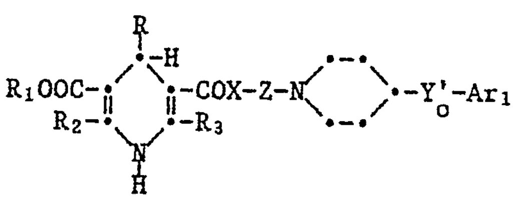

(VII)

worin Z die Bedeutung von Alk hat oder eine mittels Reduktion in die Gruppe Alk überführbare Gruppe darstellt, $Y_o'$ die Bedeutung von Y hat, oder eine mittels Reduktion in die der Bedeutung von Y entsprechende Alkylengruppe überführbare Gruppe darstellt, wobei mindestens eine der Gruppen Z und $Y_o'$ eine mittels Reduktion in die Gruppe Alk bzw. in die der Gruppe Y entsprechende Alkylengruppe überführbare Gruppe darstellt, und die andere Alk oder die Gruppe Y bedeutet, reduziert, wobei die Ausgangsstoffe der Formel II bis VII, sofern sie salzbildende Eigenschaften aufweisen, auch in Form ihrer Salze verwendet werden können, und die Gruppen R, $R_1$, $R_2$, $R_3$, X, Y, $Ar_1$ und Alk die unter der Formel I angegebenen Bedeutungen haben, und, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I in ein Salz umwandelt, und/oder, wenn erwünscht, ein erhaltenes Racematgemisch in die reinen Racemate bzw. Diastereomeren und/oder ein erhaltenes Racemat in die optischen Antipoden auftrennt.

Patentansprüche für die Vertragsstaaten AT, ES, GR

1. Verfahren zur Herstellung von Verbindungen der Formel

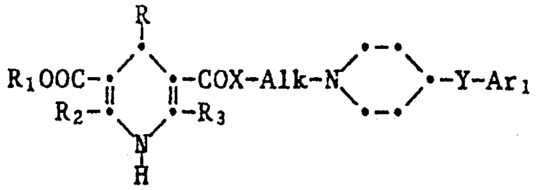

(I),

worin R einen carbocyclischen oder heterocyclischen Arylrest bedeutet, $R_1$ Niederalkyl darstellt, eine der Gruppen $R_2$ und $R_3$ für Niederalkyl und die andere für Niederalkyl, Cyano oder Amino steht X Sauerstoff oder die Gruppe -NH- darstellt, und Alk durch ein carbocyclisches Aryl substituiertes Alkylen bedeutet, das die Gruppe X vom Ringstickstoffatom durch mindestens zwei Kohlenstoffatome trennt, Y für Alkylen oder die Gruppen -CH(OH)- oder -C(=O)-steht oder eine einfache Bindung bedeutet, und $Ar_1$ einen monocyclischen Aryl- oder Heteroarylrest darstellt, und ihrer Salze davon, dadurch gekennzeichnet, dass man a) eine Verbindung der Formel

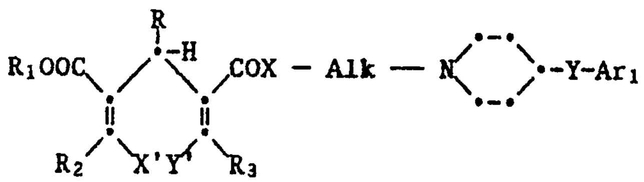

(II),

worin einer der Rest X' und Y' für die Gruppe der Formel $-NH_2$ steht und der andere Hydroxy oder die Gruppe der Formel $-NH_2$ bedeutet, oder ein Tautomeres davon oder ein entsprechendes Tautomerengemisch ringschliesst, oder

b) eine Verbindung der Formel R-CHO (III) oder ein reaktionsfähiges funktionelles Derivat davon mit einer Verbindung der Formel

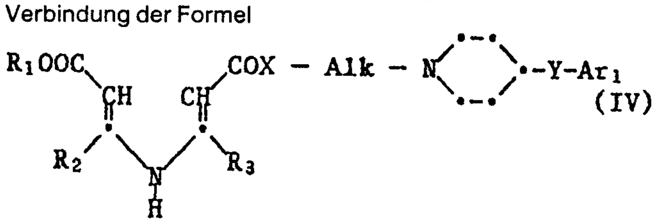

(IV)

oder einem Tautomeren davon oder einem entsprechenden Tautomerengemisch umsetzt, oder

c) in einer Verbindung der Formel

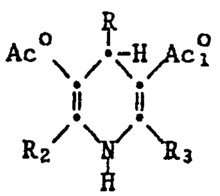

(V),

in welcher einer der Rest $Ac^o$ und $Ac^o_1$ eine in die Gruppe $-COOR_1$ bzw. in den Rest der Formel

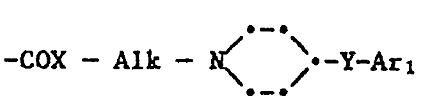

(Va)

überführbare Gruppe bedeutet, und der andere die Gruppe $-COOR_1$ oder die Gruppe der Formel Va oder einen in die Gruppe $-COOR_1$ bzw. die Gruppe der Formel Va überführbaren Rest bedeutet, den Rest $Ac^o$ in die Gruppe $-COOR_1$ und/oder den Rest $Ac^o_1$ in einen Rest der Formel Va überführt, oder

d) eine Verbindung der Formel

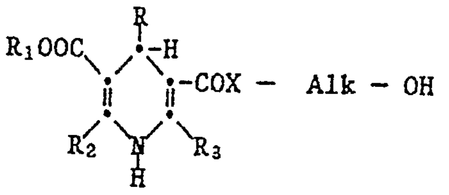

(VI)

oder einen reaktionsfähigen Ester davon, mit einer Verbindung der Formel

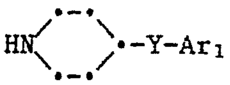

(VIa)

umsetzt, oder
e) eine Verbindung der Formel

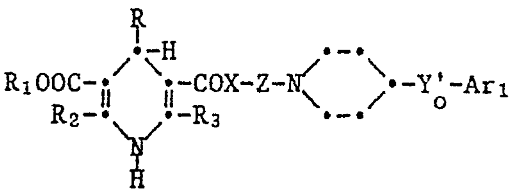

(VII)

worin Z die Bedeutung von Alk hat oder eine mittels Reduktion in die Gruppe Alk überführbare Gruppe darstellt, $Y_o'$ die Bedeutung von Y hat, oder eine mittels Reduktion in die der Bedeutung von Y entsprechende Alkylengruppe überführbare Gruppe darstellt, wobei mindestens eine der Gruppen Z und $Y_o'$ eine mittels Reduktion in die Gruppe Alk bzw. in die der Gruppe Y entsprechende Alkylengruppe überführbare Gruppe darstellt, und die andere Alk oder die Gruppe Y bedeutet, reduziert, wobei die Ausgangsstoffe der Formel II bis VII, sofern sie salzbildende Eigenschaften aufweisen, auch in Form ihrer Salze verwendet werden können, und die Gruppen R, $R_1$, $R_2$, $R_3$, X, Y, $Ar_1$ und Alk die unter der Formel I angegebenen Bedeutungen haben, und, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I in ein Salz umwandelt, und/oder, wenn erwünscht, ein erhaltenes Racematgemisch in die reinen Racemate bzw. Diastereomeren und/oder ein erhaltenes Racemat in die optischen Antipoden auftrennt.

2. Verfahren gemäss Anspruch 1 zur Herstellung von Verbindungen der Formel I, in welcher R für einen mono- oder bicyclischen, carbocyclischen Arylrest oder für einen fünf- oder sechsgliedrigen, ein bis und mit vier Ringstickstoffatome, ein Ringsauerstoff- oder Ringschwefelatom, oder ein oder zwei Ringstickstoffatome zusammen mit einem Ringsauerstoff- oder einem Ringschwefelatom als Ringglieder enthaltenden, monocyclischen Heteroarylrest steht, der über ein Ringkohlenstoffatom mit dem Kohlenstoffatom der 4-Stellung des 1,4-Dihydropyridinrings verbunden ist, und der gegebenenfalls einen ankondensierten Benzoring enthält, und insbesondere Phenyl, Naphthyl, Pyrryl, Pyrazolyl, Imidazolyl, Triazolyl, Tetrazolyl, Furyl, Thienyl, Isoxazolyl, Oxazolyl, Oxadiazolyl, Isothiazolyl, Thiazolyl, Thiadiazolyl, Pyridyl, Pyridazinyl, Pyrimidinyl, Pyrazinyl, Triazinyl, Indolyl, Isoindolyl, Benzimidazolyl, Benzofuranyl, Benzofurazanyl, Benzothienyl, Benzthiazolyl, 2,1,3-Benzthiadiazolyl, Chinolinyl oder Isochinolinyl bedeutet, wobei in diesen Resten Ringkohlenstoffatome gegebenenfalls durch Niederalkyl, Niederalkenyl, Niederalkinyl, Niederalkylen, Cycloalkyl, Phenyl und/oder Phenylniederalkyl, wobei Niederalkyl, Phenyl oder Phenylniederalkyl gegebenenfalls Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkylcarbamoyl, N,N-Diniederalkyl-carbamoyl und/oder Cyan, und cyclische Reste auch Niederalkyl, das seinerseits wie angegeben substituiert sein kann, als Substituenten enthalten können, und/oder durch Hydroxy, Niederalkoxy, Niederalkoxyniederalkoxy, Halogenniederalkoxy, Niederalkylendioxy, Niederalkanoyloxy, Halogen, Nitro, Amino, N-Niederalkyl-amino, N,N-Diniederalkylamino, N-Niederalkyl-N-phenylniederalkyl-amino, Niederalkylenamino, Oxaniederalkylenamino, Thianiederalkylenamino und/oder Azaniederalkylenamino, worin das Azastickstoffatom durch Niederalkyl, Phenyl oder Phenylniederalkyl substituiert sein kann, wobei diese Reste Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkyl-carbamoyl und/oder Cyan, die cyclischen Reste auch Niederalkyl als Substituenten enthalten können, und/oder durch Niederalkanoylamino, Azido, Nieder alkanoyl, Carboxy, Niederalkoxycarbonyl, Carbamoyl, N-Niederalkylcarbamoyl, N,N-Diniederalkyl-carbamoyl, Cyan, Sulfo, Aminosulfonyl, N-Niederalkylaminosulfonyl, N,N-Di-

niederalkylaminosulfonyl, Niederalkylthio, Halogenniederalkylthio, Niederalkylsulfinyl und/oder Niederalkylsulfonyl, und/oder Ringstickstoffatome gegebenenfalls durch Niederalkyl, das als Substituenten gegebenenfalls Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkylcarbamoyl, N,N-Diniederalkylcarbamoyl oder Cyan enthalten kann, oder durch Hydroxy oder Oxido substituiert sein können, $R_1$ Niederalkyl ist, einer der Reste $R_2$ und $R_3$ für Niederalkyl und der andere für Niederalkyl, Cyano oder Amino steht, X Sauerstoff oder die Gruppe -NH- bedeutet, und Alk durch eine carbocyclisches Aryl , z.B. durch gegebenenfalls, etwa wie nachfolgend für carbocyclisches Aryl $Ar_1$ angegeben, substituiertes Phenyl oder Naphthyl, substituiertes Alkylen darstellt, das die Gruppe X vom Ringstickstoffatom durch 2 bis 8 Kohlenstoffatome trennt, Y für Alkylen mit 1 bis 6 Kohlenstoffatomen oder für die Gruppen -CH(OH)- oder -C( = O)- steht oder eine einfache Bindung bedeutet, und $Ar_1$ einen monocyclischen carbocyclischen Aryl- oder Heteroarylrest darstellt, und insbesondere Phenyl, Naphthyl, Pyrryl, Furyl, Thienyl oder Pyridyl bedeutet, wobei in diesen Resten Ringkohlenstoffatome gegebenenfalls durch Niederalkyl, Niederalkenyl, Niederalkinyl, Niederalkylen, Cycloalkyl, Phenyl und/oder Phenylniederalkyl substituiert sind, wobei Niederalkyl, Phenyl oder Phenylniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkyl-carbamoyl und/oder Cyan, und cyclische Rest auch Niederalkyl, das seinerseits wie angegeben substituiert sein kann, enthalten können, und/oder durch Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Nitro, Amino, N-Nieder alkyl-amino, N,N-diniederalkylamino, N-Niederalkyl-N-phenylniederalkyl-amino, Niederalkylenamino, Oxoniederalkylenamino, Thianiederalkylenamino und/oder Azaniederalkylenamino, worin das Azastickstoffatom durch Niederalkyl, Phenyl oder Phenylniederalkyl substituiert sein kann, wobei diese Reste Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkyl-carbamoyl und/oder Cyan, die cyclischen Reste auch Niederalkyl als Substituenten enthalten können und/oder durch Niederalkanoylamino, Azido, Niederalkanoyl, Carboxy, Niederalkoxycarbonyl, Carbamoyl, N-Niederalkyl-carbamoyl, N,N-Diniederalkyl-carbamoyl, Cyan, Sulfo, Aminosulfonyl, N-Niederalkylaminosulfonyl, N,N-Diniederalkylaminosulfonyl, Niederalkylthio, Niederalkylsulfinyl und/oder Niederalkylsulfonyl, und/oder Ringstickstoffatome gegebenenfalls durch Niederalkyl, das als Substituenten gegebenenfalls Hydroxy, Niederalkoxy, Niederalkoxyniederalkoxy, Halogenniederalkoxy, Niederalkenyloxy, Halogenniederalkenyloxy, Niederalkinyloxy, Niederalkylendioxy, Niederalkanoyloxy, Halogen, Carboxy, Niederalkoxycarbonyl, Carbamoyl, N-Niederalkylcarbamoyl, N,N-Diniederalkyl-carbamoyl oder Cyan als Substituenten enthalten kann, oder durch Hydroxy oder Oxido substituiert sein können, und ihrer Salze, dadurch gekennzeichnet, dass man von entsprechenden Verbindungen der Formeln II, III und IV, V, VI und VIa, VII und I ausgeht.

3. Verfahren gemäss Anspruch 1 zur Herstellung von Verbindungen der Formel I, worin R und $Ar_1$ jeweils für Phenyl oder Naphthyl stehen, das gegebenenfalls durch Niederalkyl, Phenyl und/oder Phenylniederalkyl, wobei solche Reste ihrerseits Hydroxy, Niederalkoxy, Halogenniederalkoxy, Niederalkylendioxy, Halogen, Carboxy, Niederalkoxycarbonyl und/oder Cyan, die cyclischen Reste auch Niederalkyl als Substituenten enthalten können, und/oder durch Hydroxy, Niederalkoxy, Halogenniederalkoxy, Niederalkenyloxy, Halogenniederalkenyloxy, Niederalkylendioxy, Halogen, Nitro, Amino, N-Niederalkylamino, N,N-Diniederalkylamino, Niederalkanoylamino, Carboxy, Niederalkoxy carbonyl, Carbamoyl, Cyan, Sulfo, Aminosulfonyl, N-Niederalkylaminosulfonyl, N,N-Diniederalkylaminosulfonyl, Niederalkylthio, Niederalkylsulfinyl und/oder Niederalkylsulfonyl substituiert ist, oder R und $Ar_1$ jeweils für über ein Ringkohlenstoffatom gebundenes Pyrryl, Furyl, Thienyl, Pyridyl, 1-Oxido-pyridyl oder Imidazolyl, R auch für Benzofurazanyl steht, wobei solche Rest gegebenenfalls, wie für einen Phenyl- oder Naphthylrest R bzw. $Ar_1$ angegeben, substituiert sind, und insbesondere Niederalkyl, Niederalkoxy, Halogen und/oder gegebenenfalls durch Niederalkyl, Niederalkoxy, Halogen und/oder Nitro substituiertes Phenyl als Substituenten enthalten können, $R_1$ Niederalkyl ist, eine der Gruppen $R_2$ und $R_3$ Niederalkyl ist und die andere Niederalkyl oder Cyano darstellt, X Sauerstoff oder die Gruppe -NH- bedeutet, und Alk durch ein gegebenenfalls durch Niederalkyl, Niederalkoxy, Hydroxy, Halogenniederalkyl, Halogen, Cyan und/oder Nitro substituiertes Phenyl oder Naphthyl substituiertes Alkylen mit 2 bis 6 Kohlenstoffatomen steht, das die Gruppe X vom Ringstickstoffatom durch 2 bis 6 Kohlenstoffatome trennt und Y Alkylen mit 1 bis 4 Kohlenstoffatomen oder die Gruppen -CH(OH)- oder -C( = O) bedeutet, und ihrer Salze, dadurch gekennzeichnet, dass man von entsprechenden Verbindungen der Formeln II, III und IV, V, VI und VIa, VII und I ausgeht.

4. Verfahren gemäss Anspruch 1 zur Herstellung von Verbindungen der Formel I, worin R und $Ar_1$ jeweils für Phenyl stehen, das gegebenenfalls durch Niederalkyl, Hydroxy, Niederalkoxy, Halogenniederalkoxy, Halogenniederalkenyloxy, Niederalkylendioxy, Halogen, Trifluormethyl, Nitro, Niederalkanoylamino, gegebenenfalls durch gegebenenfalls verestertes oder veräthertes Hydroxy substituiertes Phenyl oder Phenylniederalkyl, und/oder Cyan substituiert ist, wobei ein Phenylrest R bzw. $Ar_1$ einen oder mehrere, gleiche oder verschiedene Substituenten aufweisen kann, oder R und $Ar_1$ jeweils Pyridyl, Furyl, 1-Oxido-pyridyl, oder Thienyl, R auch Benzofurazanyl, z.B. 4-Benzofurazanyl, bedeutet, das durch

Niederalkyl oder Halogen substituiert sein kann, $R_1$, $R_2$ und $R_3$ jeweils Niederalkyl bedeuten, X für die Gruppe -NH-, insbesondere jedoch für Sauerstoff steht, Alk durch ein gegebenenfalls Niederalkyl, Niederalkoxy, Hydroxy, Halogen niederalkyl, Halogen, Cyan und/oder Nitro enthaltendes Phenyl substituiertes Alkylen mit 2 bis 4 Kohlenstoffatomen bedeutet, wobei der gegebenenfalls substituierte Phenylrest vorzugsweise an das an die Gruppe X benachbarte Kohlenstoffatome gebunden ist, und Alk die Gruppe X vom Ringstickstoffatom durch 2 bis 4 Kohlenstoffatome trennt, Y für Alkylen mit 1 bis 3 Kohlenstoffatomen oder die Gruppen -CH(OH)- oder insbesondere -C(=O)- steht, und ihrer Salze, dadurch gekennzeichnet, dass man von entsprechenden Verbindungen der Formeln II, III und IV, V, VI und VIa, VII und I ausgeht.

5. Verfahren gemäss Anspruch 1 zur Herstellung von Verbindungen der Formel I, worin R für unsubstituiertes oder vorzugsweise durch Niederalkyl, z.B. Methyl, Niederalkoxy, z.B. Methoxy, Halogenniederalkoxy, z.B. Difluormethoxy, oder Halogenniederalkenyloxy, worin Halogen eine Atomnummer bis und mit 35 hat und insbesondere Fluor oder Chlor ist, z.B. 1,1-Difluorvinyloxy, Halogen mit einer Atomnummer bis und mit 35, insbesondere Chlor sowie Fluor, Trifluormethyl, Nitro und/oder Cyan mono- oder di-substituiertes Phenyl steht, wobei Substituenten die 2- und/oder 3-Stellung einnehmen, $R_1$ Niederalkyl z.B.Methyl oder Aethyl, und $R_2$ und $R_3$ Niederalkyl, insbesondere Methyl, bedeuten, X für die Gruppe -NH-, insbesondere jedoch für Sauerstoff steht, Alk durch ein durch Niederalkyl, Niederalkoxy, Hydroxy, Halogenniederalkyl, Halogen, Cyan und/oder Nitro substituiertes, insbesondere aber durch ein unsubstituiertes Phenyl substituiertes Alkylen mit 1 bis 3 Kohlenstoffatomen darstellt, wobei der gegebenenfalls substituierte Phenylrest an das der Gruppe X benachbarte Kohlenstoffatom gebunden ist, und Alk die Gruppe X vom Ringstickstoffatom durch 2 bis 3 Kohlenstoffatome trennt, Y für Alkylen mit 1 bis 3 Kohlenstoffatomen oder insbesondere die Gruppe -C(=O)- steht, und $Ar_1$ gegebenenfalls durch Halogen mit einer Atomnummer bis und mit 35, z.B. Fluor oder Chlor, substituiertes Phenyl oder Pyridyl bedeutet, und ihrer Salze, dadurch gekennzeichnet, dass man von entsprechenden Verbindungen der Formeln II, III und IV, V, VI und VIa, VII und I ausgeht.

6. Verfahren gemäss Anspruch 1 zur Herstellung der Verbindungen der Formel I, worin R durch Halogenniederalkoxy, insbesondere mit einer Atomnummer bis und mit 35 und mit bis 4 C-Atomen, wie Difluormethoxy, Niederalkylendioxy, insbesondere mit bis und mit 3 C-Atomen, wie Methylendioxy, Halogen, insbesondere mit einer Atomnummer bis und mit 35, wie Chlor, Trifluormethyl oder Nitro mono-oder disubstituiertes Phenyl, wobei Substituenten die 2- und/oder 3-Stellung einnehmen, Benzofurazanyl, insbesondere 4-Benzofurazanyl, oder 1-Oxido-pyridyl, wie 1-Oxido-3-pyridyl, bedeutet, $R_1$, $R_2$ und $R_3$ unabhängig voneinander Niederalkyl, insbesondere mit bis und mit 4 C-Atomen, wie Methyl, bedeuten, X für Sauerstoff steht, Alk durch ein gegebenenfalls Niederalkyl, Niederalkoxy, jeweils insbesondere mit bis und mit 4 C-Atomen, wie Methyl oder Methoxy, Halogenniederalkyl, insbesondere mit einer Atomnummer bis und mit 35 und mit bis und mit 4 C-Atomen, wie Trifluormethyl, Halogen, insbesondere mit einer Atomnummer bis und mit 35, wie Chlor, oder Cyan substituiertes Phenyl enthaltendes Alkylen mit 2 bis 4 C-Atomen bedeutet, wobei Alk die Gruppe X vom Ringstickstoffatom durch 2 bis 4 C-Atome trennt, Y für Alkylen mit 1 bis 3 C-Atomen, die Gruppen -CH(OH)- oder insbesondere -C(=O)- oder eine Bindung steht, und $Ar_1$ gegebenenfalls durch Halogen, insbesondere mit einer Atomnummer bis und mit 35, wie Chlor, substituiertes Phenyl oder Thienyl, wie 2-Thienyl, bedeutet, und ihrer Salze, dadurch gekennzeichnet, dass man von entsprechenden Verbindungen der Formeln II, III und IV, V, VI und VIa, VII und I ausgeht.

7. Verfahren gemäss Anspruch 1 zur Herstellung von Verbindungen der Formel I, worin R durch Halogen mit einer Atomnummer bis und mit 35, wie Chlor, Trifluormethyl oder Nitro mono- oder disubstituiertes Phenyl steht, wobei Substituenten bevorzugt die 2- oder 3-Stellung einnehmen, $R_1$, $R_2$ und $R_2$ unabhängig voneinander Niederalkyl mit bis und mit 4 C-Atomen, wie Methyl oder Aethyl, bedeuten, X für Sauerstoff steht, Alk ein durch Niederalkyl, Niederalkoxy jeweils mit bis und mit 4 C-Atomen, wie Methyl oder Methoxy, Halogenniederalkyl mit einer Atomnummer bis und mit 35 und mit bis und mit 4 C-Atomen, wie Trifluormethyl, Halogen mit einer Atomnummer bis und mit 35, wie Chlor, oder Cyan substituiertes oder unsubstituiertes Phenyl substituiertes Alkylen mit 2 bis und mit 6 C-Atomen, bedeutet, wobei der gegebenenfalls substituierte Phenylrest vorzugsweise an das an die Gruppe X benachbarte C-Atom gebunden ist und die Gruppe X vom Ringstickstoffatom durch mindestens zwei C-Atome getrennt wird, Y für Alkylen mit 1 bis 3 C-Atomen, die Gruppen -CH(OH)- oder -(C=O), ferner eine Bindung steht, und $Ar_1$ unsubstituiertes oder durch Halogen mit einer Atomnummer bis und mit 35, wie Fluor, substituiertes Phenyl oder Thienyl, wie 2-Thienyl, bedeutet, und ihrer Salze, dadurch gekennzeichnet, dass man von entsprechenden Verbindungen der Formeln II, III und IV, V, VI und VIa, VII und I ausgeht.

8. Verfahren gemäss Anspruch 1 zur Herstellung von Verbindungen der Formel I, worin R für 2-, 3-Nitrophenyl, 2-, 3-Trifluormethylphenyl oder 2,3-Dichlorphenyl steht, $R_1$, $R_2$ und $R_3$ unabhängig voneinander jeweils Niederalkyl mit bis und mit 4 C-Atomen, insbesondere Methyl, bedeuten, X für Sauerstoff steht, Alk ein durch Niederalkyl mit bis und mit 4 C-Atomen, wie Methyl, Niederalkoxy mit bis und mit 4 C-Atomen, wie Methoxy, Halogenniederalkyl mit einer Atomnummer bis und mit 35 und mit bis und mit 4 C-Atomen, wie Trifluormethyl, Halogen mit einer Atomnummer bis und mit 35, wie Chlor, oder Cyano monosubstituiertes oder unsubstituiertes Phenyl substituiertes Alkylen mit 2 bis und mit 6 C-Atomen bedeutet und wobei die Gruppe X vom Ringstickstoffatom durch mindestens zwei C-Atome

getrennt wird, Y für Methylen, Hydroxymethylen, die Gruppe -C(=O)- oder eine Bindung steht, und $Ar_1$ unsubstituiertes oder durch Halogen mit einer Atomnummer bis und mit 35, wie Fluor, monosubstituiertes Phenyl oder Thienyl, wie 2-Thienyl, bedeutet, und ihrer Salze, dadurch gekennzeichnet, dass man von entsprechend Verbindungen der Formeln II, III und IV, V, VI und VIa, VII und I ausgeht.

9. Verfahren gemäss Anspruch 1 zur Herstellung von Verbidungen der Formel I, worin R für durch Halogen mit einer Atomnummer bis und mit 35, besonders Fluor oder Chlor, mono- oder disubstituiertes oder durch Trifluormethyl, Nitro oder Cyan monosubstituiertes Phenyl steht, wobei Substituenten die 2- und/oder 3-Stellung einnehmen, $R_1$ Niederalkyl, z.B. Methyl oder Aethyl, und $R_2$ und $R_3$ jeweils Niederalkyl, in erster Linie Methyl, bedeuten, X für Sauerstoff steht, Alk durch ein durch Niederalkyl, z.B. Methyl, Niederalkoxy, z.B. Methoxy, Trifluormethyl, Cyan und/oder Nitro substituiertes, insbesondere aber auch ein unsubstituiertes, Phenyl substituiertes Alkylen mit 2 bis 3 Kohlenstoffatomen darstellt, wobei der gegebenenfalls substituierte Phenylrest an das der Gruppe X benachbarte Kohlenstoffatom gebunden ist, und Alk die Gruppe X vom Ringstickstoffatom durch 2 bis 3 Kohlenstoffatome trennt, Y für Alkylen mit 1 bis 2 Kohlenstoffatomen oder insbesondere die Gruppe -C(=O)- steht, und $Ar_1$ gegebenenfalls, insbesondere in 4-Stellung, durch Halogen mit einer Atomnummer bis und mit 35, z.B. Fluor, substituiertes Phenyl bedeutet, und ihrer Salze, dadurch gekennzeichnet, dass man von entsprechenden Verbindungen der Formeln II, III und IV, V, VI und VIa, VII und I ausgeht.

10. Verfahren gemäss Anspruch 1 zur Herstellung von Verbindungen der Formel I, worin R 3-Nitrophenyl oder 2-Trifluormethylphenyl bedeutet, $R_1$ Methyl oder Aethyl bedeutet, $R_2$ und $R_3$ Methyl bedeuten, X für Sauerstoff steht, Alk 1-Phenyläthyl bedeutet, Y für die Gruppe -C(=O)- steht und $Ar_1$ gegebenenfalls in 4-Stellung durch Fluor substituiertes Phenyl bedeutet, und ihrer Salze, dadurch gekennzeichnet, dass man von entsprechenden Verbindungen der Formeln II, III und IV, V, VI und VIa, VII und I ausgeht.

11. Verfahren gemäss Anspruch 1 zur Herstellung von 2,6-Dimethyl-4-(3-nitrophenyl)-1,4-dihydropyridin-3-carbonsäuremethylester-5-carbonsäure-[2-[4-(p-fluorbenzoyl)-piperidin-1-yl]-1-phenyl]-äthylester oder einem Salz davon, dadurch gekennzeichnet, dass man von entsprechenden Verbindungen der Formeln II, III und IV, V, VI und VIa, VII und I ausgeht.

12. Verfahren gemäss Anspruch 1 zur Herstellung von 2,6-Dimethyl-4-(2-trifluormethyl-phenyl)-1,4-dihydropyridin-3-carbonsäuremethylester-5-carbonsäure-[2-[4-(p-fluorbenzoyl)-piperidin-1-yl]-1- phenyl]-äthylester oder einem Salz davon, dadurch gekennzeichnet, dass man von entsprechenden Verbindungen der Formeln II, III, IV, V, VI und VIa, VII und I ausgeht.

13. Verfahren gemäss Anspruch 1 zur Herstellung von 1,4-Dihydro-2,6-dimethyl-4-(3-nitrophenyl)-pyridin-3-carbonsäuremethylester-5-[2-(4-phenylpiperidin-1-yl)-1-phenyl]-äthylester oder einem Salz davon, dadurch gekennzeichnet, dass man von entsprechenden Verbindungen der Formeln II, III und IV, V, VI und VIa, VII und I ausgeht.

14. Verfahren gemäss Anspruch 1 zur Herstellung von 2,6-Dimethyl-4-(3-nitrophenyl)-1,4-dihydropyridin-3-carbonsäuremethylester-5-carbonsäure-[6-[4-(p-fluorbenzoyl)-piperidin-1-yl]-1-phenyl]-n-hexylester, 2,6-Dimethyl-4-(3-nitrophenyl)-1,4-dihydropyridin-3-carbonsäuremethylester-[2-[4-(p-chlorbenzoyl)-piperidin-1-yl]-1-phenyl]-äthylester, 2,6-Dimethyl-4-(3-nitrophenyl)-1,4-dihydropyridin-3-carbonsäuremethylester-5-carbonsäure-[2-[4-(2-thenoyl)-piperidin-1-yl]-1-phenyl]-äthylester, 2,6-Dimethyl--4-(3-nitrophenyl)-1,4-dihydropyridin-3-carbonsäuremethylester-5-carbonsäure-[2-[4-(p-fluorphenyl)-piperidin-1-yl]-1-phenyl]-äthylester, 2,6-Dimethyl-4-(3-nitrophenyl)-1,4-dihydropyridin-3-carbonsäuremethylester-5-carbonsäure-[ 2-[4-(p-fluorphenyl)-hydroxymethylenpiperidin-1-yl]-1-phenyl]-äthylester, 2,6-Dimethyl-4-(3-nitrophenyl)-1,4-dihyrdopyridin-3-carbonsäuremethylester-5-carbonsäure[2-[4-(p-fluorbenzyl)-piperidin-1-yl]-1-phenyl]-äthylester, 2,6-Dimethyl-4-(3-nitrophenyl)-1,4-dihydropyridin-3-carbonsäuremethylester-5-carbonsäure-[2-[4-(p-fluorbenzoyl)-piperidin-1-yl]-1-(p-methoxyphenyl)]-äthylester, 2,6-Dimethyl-4-(3-nitrophenyl)-1,4-dihydropyridin-3-carbonsäuremethylester-5-carbonsäure--[2-[4-(p-fluorbenzoyl)-piperidin-1-yl]-1-(p-cyanophenyl)]-äthylester, 2,6-Dimethyl-4-(3-nitrophenyl)-1,4-dihydropyridin-3-carbonsäuremethylester-5-carbonsäure-[2-[4-(p-fluorbenzoyl)-piperidin--1-yl]-1-(p-methylphenyl]-äthylester, 2,6-Dimethyl-4-(3-nitrophenyl)-1,4-dihydropyridin-3-carbonsäuremethylester-5-carbonsäure-[2-[4-(p-fluorbenzoyl)-piperidin-1-yl]-1-(p-chlorphenyl)]-äthylester, 2,6-Dimethyl-4-(3-nitrophenyl)-1,4-dihydropyridin-3-carbonsäuremethyl ester-5-carbonsäure-[2-[4-(p-fluorbenzoyl)-piperidin-1-yl]-1-(p-trifluormethylphenyl)]-äthylester, 2,6-Dimethyl-4-(3-nitrophenyl)-1,4-dihydropyridin-3-carbonsäuremethylester-5-carbonsäure-[3-[4-(p-fluorbenzoyl)-piperidin-1-yl]-1-phenyl]-propylester, 2,6-Dimethyl-4-(3-nitrophenyl)-1,4-dihydropyridin-3-carbonsäuremethylester-5-carbonsäure--[4-[4-(p-fluorbenzoyl)-piperidin-1-yl]-1-phenyl]-butylester, 2,6-Dimethyl-4-(3-nitrophenyl)-1,4-dihydropyridin-3-carbonsäuremethylester-5-carbonsäure-[2-[4-(p-fluorbenzoyl)-piperidin-1-yl]-2-phenyl]-äthylester, 2,6-Dimethyl-4-(3-nitrophenyl)-1,4-dihydropyridin-3-carbonsäuremethylester-5-carbonsäure--[3-[4-(p-fluorbenzoyl)-piperidin-1-yl]-3-phenyl]-propylester, 2,6-Dimethyl-4-(3-trifluormethylphenyl)-1,4-dihydropyridin-3-carbonsäuremethylester-5-carbonsäure-[2-[4-(p-fluorbenzoyl)-piperidin--1-yl]-1-phenyl]-äthylester, 2,6-Dimethyl-4-(2,3-dichlorphenyl)-1,4-dihydropyridin-3-carbonsäuremethylester-5-carbonsäure-[2-[4-(p-fluorbenzoyl)-piperidin-1-yl]-1-phenyl]-äthylester, 2,6-Dimethyl-4-(2-nitrophenyl)-1,4-dihydropyridin-3-carbonsäuremethylester-5-carbonsäure-[2-[4-(p-fluorbenzoyl)-piperidin-1-yl]-1-phenyl ]-äthylester und einem Salz davon, dadurch gekennzeichnet, dass man von entsprechenden Verbindungen der Formeln II, III und IV, V, VI und VIa, VII und I ausgeht.

15. Verfahren zur Herstellung von pharmazeutischen Präparaten, dadurch gekennzeichnet, dass man

eine Verbindung gemäss einer der Ansprüche 1-14 in freier Form oder in Form eines pharmazeutisch verwendbaren, nicht-toxischen Salzes, gegebenenfalls unter Beimischung von üblichen Hilfs- und Trägerstoffen, zu pharmazeutischen Präparaten verarbeitet.